(19) Europäisches Patentamt
European Patent Office
Office européen des brevets

(11) EP 4 481 027 A1

(12) EUROPEAN PATENT APPLICATION

(43) Date of publication:
**25.12.2024 Bulletin 2024/52**

(21) Application number: **23180130.9**

(22) Date of filing: **19.06.2023**

(51) International Patent Classification (IPC):
***C11D 3/386*** *(2006.01)*

(52) Cooperative Patent Classification (CPC):
**C12N 9/24; C11D 3/386; C11D 3/38636; C12N 9/2402; C12N 9/88; C12Y 302/01006; C12Y 302/01039; C12Y 302/01058; C12Y 402/02;**
C12N 9/50; C12Y 302/01001

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **The Procter & Gamble Company**
**Cincinnati, OH 45202 (US)**

(72) Inventors:
- **BELL-RUSIEWICZ, Katarzyna Dorota**
  **Newcastle upon Tyne, NE12 9BZ (GB)**
- **BLACK, Gary William**
  **Newcastle upon Tyne, NE1 8ST (GB)**
- **BROWN, Nicola Louise**
  **Newcastle upon Tyne, NE1 8ST (GB)**
- **LANT, Neil Joseph**
  **Newcastle upon Tyne, NE12 9BZ (GB)**
- **MALEKPOUR, Adam Kurosh**
  **Newcastle upon Tyne, NE12 9BZ (GB)**
- **MORALES-GARCIA, ANA L.**
  **Newcastle upon Tyne, NE12 9BZ (GB)**
- **MUNOZ, Jose Luis Munoz**
  **Newcastle upon Tyne, NE1 8ST (GB)**
- **YAU, Hamish Chun Lam**
  **United Kingdom, NE12 9BZ (GB)**

(74) Representative: **P&G Patent Belgium UK**
**N.V. Procter & Gamble Services Company S.A.**
**Temselaan 100**
**1853 Strombeek-Bever (BE)**

Remarks:
The complete document including Reference Table(s) and the Sequence Listing(s) can be downloaded from the EPO website

# (54) CLEANING COMPOSITIONS CONTAINING ENZYMES

(57) The invention relates to cleaning compositions, preferably for automatic dishwashing and/or laundry, comprising an enzyme endogenous to *Alkalitalea saponilacus* or a variant thereof. The invention also relates to methods of cleaning using the composition of the invention and the use of the enzyme in cleaning compositions.



Processed by Luminess, 75001 PARIS (FR)

## Description

REFERENCE TO A SEQUENCE LISTING

This application contains a Sequence Listing in computer readable form. The computer readable form is incorporated herein by reference.

FIELD OF THE INVENTION

The present invention relates to cleaning compositions, preferably for automatic dishwashing and/or laundry, comprising an enzyme endogenous to *Alkalitalea saponilacus* or a variant thereof. The invention also relates to methods of cleaning using the composition of the invention and the use of the enzyme in cleaning compositions.

BACKGROUND OF THE INVENTION

In cleaning applications, particularly for automatic dishwashing and laundry, stain, soil, and malodour removal can present permanent unresolved problems. There are many cleaning technologies aimed at mitigating such problems however, it is a constant challenge to provide improved efficacy and especially in an environmentally favourable manner. In automatic dishwashing and laundry washing machines these problems are compounded by the increased use of low wash temperatures (e.g., cold water) and shorter washing cycles which can reduce stain/soil/malodor removal efficacy of cleaning compositions and exacerbate problems of redeposition of soil onto surfaces during the washing process.

Enzymes are routinely used in cleaning compositions. Many enzymes perform better in acidic or neutral conditions. Often, enzyme stability in product and performance is not optimum in alkaline compositions. There is a constant search for enzymes with improved stability and improved performance in alkaline cleaning compositions. Thus, it is an object of the present invention to provide a cleaning composition, particularly for automatic dishwashing and/or laundry with improved soil and/or stain and/or malodor removal.

It is also an object of the present invention to provide a cleaning composition, particularly for automatic dishwashing and/or laundry which can be used in a washing process, even at low temperatures and short wash times, which will remove complex soils and stains, for example to enable cosmetic removal, such as make up, mascara, etc. Other stains difficult to remove include protein-containing stains, more in particular sugary/fatty protein-containing stains, such as milk chocolate-based beverage or dessert stains.

SUMMARY OF THE INVENTION

According to the first aspect of the invention, there is provided an alkaline cleaning composition. The composition comprises an enzyme. The enzyme is endogenous to *Alkalitalea saponilacus* or a variant thereof. The composition also comprises at least one cleaning component. The cleaning component is preferably selected from a surfactant, a builder, a bleach component, a polymer, a dispersing agent, and an additional enzyme. Preferably, the composition comprises an additional enzyme. Preferably, the composition comprises a surfactant. Preferably, the composition comprises an enzyme and a surfactant. The composition can be any cleaning composition. Especially useful compositions for use herein are laundry and automatic dishwashing compositions. By "alkaline composition" is herein understood a composition having a pH above 7, preferably from 7.5 to 11, more preferably from 8 to 11, as measured in a 10% by weight aqueous composition at 25°C.

According to the second aspect of the invention, there is provided a method of cleaning a surface using the composition of the invention. The surface can be a hard surface, such as a dishware surface or a soft surface, such as a fabric. In the case of a method of treating a fabric, the method comprises contacting a fabric with an aqueous wash liquor comprising the composition of the invention. Preferably the aqueous wash liquor comprises anionic surfactant in an amount from 0.05g/l to 5g/l, preferably from 0.01g/l to 3g/l. The fabric is typically contacted with the aqueous wash liquor at a temperature of 60°C or less, preferably at a temperature of 40°C or less or 35°C or less, most preferably at a temperature of 30°C or less or even 25°C or less; and (iii) optionally rinsing the surface. The compositions and methods herein are particularly useful for treating any dishware or fabric surface. Fabric surfaces include synthetic or natural, including cotton, wool, silk, polyester, nylon, elastane or mixed fabric, such as polycotton.

The composition and method described herein may also give rise to biofilm-disrupting effects.

According to the third aspect of the invention, there is provided the use of the enzyme of the invention in a cleaning composition to provide stain, soil and/or malodour removal, in particular, the use of the enzyme of the invention in a cleaning composition provides good removal of cosmetic stains, preferably from fabrics. The composition of the invention also provides good removal of sugary/fatty protein-containing stains, such as milk chocolate-based beverage or dessert stains, preferably from fabrics. The composition of the invention also provides good removal of starchy soils, such as

oatmeal, preferably from dishware.

The elements of the composition of the invention described herein apply *mutatis mutandis* to the use and method aspects of the invention.

DETAILED DESCRIPTION OF THE INVENTION

Definitions

Parent or Parent enzyme: The term "parent" or "parent enzyme" means an enzyme to which an alteration is made to produce the enzyme variants. The parent may be a naturally occurring (wild type) polypeptide or a variant thereof. For example, the parent may be any of SEQ ID NOs: 1 to 190 listed herein.

Sequence Identity: The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labelled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

Variant: The term "variant" means a polypeptide having enzyme activity comprising an alteration/mutation, i.e., a substitution, insertion, and/or deletion, at one or more (e.g., several) positions relative to the parent. A substitution means a replacement of an amino acid occupying a position with a different amino acid; a deletion means removal of an amino acid occupying a position; and an insertion means adding 1-3 amino acids adjacent to and immediately following an amino acid occupying a position.

Wild-Type Enzyme: The term "wild type" enzyme means an enzyme expressed by a naturally occurring microorganism, such as a bacterium, algae, yeast, or filamentous fungus found in nature.

The enzyme endogenous to *Alkalitalea saponilacus* or a variant thereof as defined in claim 1 is sometimes herein referred to as "the enzyme of the invention".

All percentages, ratios and proportions used herein are by weight percent of the composition, unless otherwise specified. All average values are calculated "by weight" of the composition, unless otherwise expressly indicated.

All measurements are performed at 25°C unless otherwise specified.

Cleaning Composition

The cleaning composition comprises (a) from 0.00005 to 5%, preferably from 0.0005 to 2% by weight of the composition, based on active protein, of an enzyme endogenous to *Alkalitalea saponilacus* or a variant thereof. The cleaning composition also comprises at least one cleaning component preferably selected from a surfactant, a builder, a bleach component, a polymer, a dispersing agent, and an additional enzyme. More preferably the composition comprises an additional enzyme. By "additional enzyme" is herein meant an enzyme other than an enzyme endogenous to *Alkalitalea saponilacus* or a variant thereof as described in claim 1. More preferably, the composition comprises an additional enzyme and a surfactant. In the case of a dishwashing composition, the composition comprises additional enzymes selected from amylases, proteases, and mixtures thereof and preferably a non-ionic surfactant. In the case of a laundry composition, the composition comprises additional enzymes selected from amylases, proteases, lipases, and mixtures thereof and preferably an anionic surfactant.

Benefits that may be associated to the composition of the invention include:

a) reducing or preventing soil redeposition;
b) removal of cereal containing soil, especially dried-on cereal containing soil, preferably oat flakes containing soil, especially dried-on oat flakes containing soil and/or cooked oats containing soil, and/or cooked and burned-in oats containing soil, and/or uncooked oats containing soil,
c) removal of chocolate containing soil, especially chocolate porridge oats containing soil, and/or chocolate milkshake containing soil, and/or chocolate drinks containing soil;
d) removal of cosmetics and/or personal care containing soil;

e) removal of tomato containing soil, especially tomato soup containing soil, and/or tomato sauce, such as spaghetti sauce containing soil;
f) facilitating removal of starch-containing soil in the presence of one or more amylases and/or for enhancing amylase related cleaning performance;
g) facilitating removal of protein-containing soil in the presence of one or more proteases and/or for enhancing protease related cleaning performance;
h) facilitating removal of carbohydrase-containing soil in the presence of one or more other carbohydrases and/or enhancing carbohydrase related cleaning performance;
i) reducing or removing a biofilm from an item, such as textile, preferably in a cleaning process such as laundry;
j) cleaning, e.g., deep cleaning of an item, wherein the item is a textile or a surface.

The composition of the present invention may be in the form of a composition for use in a main wash step or as pre-treatment or rinse-added cleaning composition for consumer or institutional use.

*Alkalitalea saponilacus*

Detergent enzymes have historically derived from bacterial (e.g., *Bacillus* and *Pseudomonas* spp.) and fungal strains (e.g., *Aspergillus* and *Thermomyces* spp.). For example, cleaning proteases and amylases have been derived from *Bacillus* spp., cellulases from *Bacillus* or fungi, and lipases from bacterial and fungal sources (see references below).
Flickinger, M.C. and Maurer, K.-H. (2010). Enzymes, Detergent. In Encyclopaedia of Industrial Biotechnology, M.C. Flickinger (Ed.).
Olsen, H.S. and Falholt, P. (1998), The role of enzymes in modern detergency. J Surfact Deterg, 1: 555-567.
Li XL, Zhang WH, Wang YD, Dai YJ, Zhang HT, Wang Y, Wang HK, Lu FP (2014) A high-detergent-performance, cold-adapted lipase from Pseudomonas stutzeri PS59 suitable for detergent formulation. J Mol Catal B Enzym 102: 16-24.
Extensive screening of other organisms from natural sources with environmental conditions similar to cold water detergent wash solutions have identified other examples of detergent compatible enzymes from strains such as *Pseudomonas* spp., *Yersinia enterocolitica, Acinetobacter* spp. (See references below).
Mykytczuk, N., Foote, S., Omelon, C. et al. Bacterial growth at -15 °C; molecular insights from the permafrost bacterium Planococcus halocryophilus Or1. ISME J 7, 1211-1226 (2013).
Al-Ghanayem, A.A., Joseph, B. Current prospective in using cold-active enzymes as eco-friendly detergent additive. Appl Microbiol Biotechnol 104, 2871-2882 (2020).
Ji X, Chen G, Zhang Q, Lin L (2015) Purification and characterization of an extracellular cold-adapted alkaline lipase produced by psychrotrophic bacterium Yersinia enterocolitica strain KM1. J Basic Microbiol 55(6):718-728.
It is desirable to find alternative sources of cleaning enzymes to traditional sources such as *Bacillus* spp. It has been surprisingly found that *Alkalitalea saponilacus* produces enzymes that are well suited to be used in cleaning compositions and therefore provide a promising source of many detergent enzyme classes. Characterization of the strain can be found in Zhao B, Chen S. Alkalitalea saponilacus gen. nov., sp. nov., an obligately anaerobic, alkaliphilic, xylanolytic bacterium from a meromictic soda lake. Int J Syst Evol Microbiol 2012; 62:2618-2623. *Alkalitalea* is a Gram-positive and obligately anaerobic genus of bacteria from the family of *Marinilabiliaceae* with one known species (*Alkalitalea saponilacus). Alkalitalea saponilacus* has been isolated from sediment-water mixture from the meromictic soda lake in Soap Lake, Washington, US.

The enzyme of the invention

The composition of the invention comprises an enzyme endogenous to *Alkalitalea saponilacus* or an enzyme that is a variant thereof. The enzyme of the invention belongs to a class selected from the group consisting of glycosyl hydrolase, polysaccharide lyase, esterase / lipase, nuclease, and protease / peptidase. Preferably, the enzyme of the invention is a glycosyl hydrolase, more preferably a laminarinase, preferably an endo-beta-1,3-glucanase (E.C. 3.2.1.39).

Glycosyl hydrolase

Glycoside hydrolases (also called glycosidases or glycosyl hydrolases) catalyze the hydrolysis of glycosidic bonds in complex sugars. Glycoside hydrolases are classified into EC 3.2.1 as enzymes catalyzing the hydrolysis of O- or S-glycosides. Glycoside hydrolases are typically named after the substrate that they act upon. Thus, glucosidases catalyze the hydrolysis of glucosides and xylanases catalyze the cleavage of the xylose based homopolymer xylan. Other examples include laminarinase,lactase, amylase, chitinase, sucrase, maltase, neuraminidase, invertase, hyaluronidas e and lysozyme. Preferred for the compositions of the inventions are laminarinases.
Beta-glucans are polysaccharides consisting of glucose units linked by beta-glycosidic bonds. Cellulose is one

type of beta-glucan, in which all the glucose units are linked by beta-1,4-glucosidic bonds. This feature results in the formation of insoluble cellulose micro-fibrils. Enzymatic hydrolysis of cellulose to glucose requires the use of endo beta-1,4-glucanases (e.g., EC 3.2.1.4), cellobiohydrolases (e.g., EC 3.2.1.91) and beta-glucosidases (e.g., EC 3.2.1.21).

Beta-glucans can also be linked by beta-1,3-glucosidic bonds (e.g., as found in the cell walls of baker's yeast, *Saccharomyces cerevisiae),* beta-1,6-glucosidic bonds as well as combinations of beta-1,3-, beta-1,4- and beta-1,6-glucosidic bonds. The combination of beta-1,3- and beta-1,4-glucosidic bonds can be found, e.g., in the soluble fibre from cereals such as oats and barley. In addition, storage polysaccharides found in algae contain 1,3-linked beta-D-glucose residues with various degrees of 1,6-branching. A subgroup of beta-glucanases, also known as laminarinases, can be classified as endo-1,3-beta-glucanases (EC 3.2.1.6 and EC 3.2.1.39) or exo-1,3-beta-glucanases (EC 3.2.1.58). Laminarinases can be used to catalyse the hydrolysis of the beta-1,3-glucosidic bonds, or beta-1,4-glucosidic bonds when the glucose residue whose reducing group is involved in the linkage to be hydrolysed is substituted at C3 to release glucose or oligosaccharides. These enzymes can act on laminarin, lichenin and cereal beta-D-glucans, but not on substrates containing only 1,4-bonds. Other beta-glucanases (e.g., EC 3.2.1.4) can, for example, perform endohydrolysis of (1,4)-beta-D-glucosidic linkages in cellulose, lichenin and cereal beta-D-glucans and will also hydrolyze 1,4-linkages in beta-D-glucans containing 1,3-linkages. Still other beta-glucanases (e.g., licheninases EC 3.2.1.73) can hydrolyze (1,4)-beta-D-glucosidic linkages in beta-D- glucans containing (1,3)- and (1,4)-bonds, but not on substrates containing only 1,3- or only 1,4-bonds.

The removal of cereal stains such as oat and barley containing stains in dish wash and laundry is a recognised problem, and there is a considerable interest in finding enzymes that can degrade the beta-glucans found therein.

Laminarinase (EC 3.2.1.6, EC 3.2.1.39, and/or EC 3.2.1.58) and especially endo-beta-1,3-glucanase (E.C. 3.2.1.39), more especially an enzyme having at least 35%, 45%, 55%, 65%, 75%, 80%, 85%, 90%, 95%, 97%, 98%, 99%, 100% sequence identity to SEQ ID NO: 1, is preferred for use in the composition of the invention. The present invention provides cleaning compositions comprising laminarinases, in particular endo-beta-1,3-glucanase (E.C. 3.2.1.39) with significant improvement of performance and/or stability under alkaline conditions.

The composition of the invention comprises a glycosyl hydrolase endogenous to *Alkalitalea saponilacus* or a variant thereof selected from the group consisting of glycosyl hydrolase having at least 35% sequence identity to SEQ ID NO: 1, at least 45% sequence identity to SEQ ID NO: 2, at least 55% sequence identity to SEQ ID NO: 3, at least 50% sequence identity to SEQ ID NO: 4, at least 60% sequence identity to SEQ ID NO: 5, at least 50% sequence identity to SEQ ID NO: 6, at least 50% sequence identity to SEQ ID NO: 7, at least 50% sequence identity to SEQ ID NO: 8, at least 40% sequence identity to SEQ ID NO: 9, at least 45% sequence identity to SEQ ID NO: 10, at least 60% sequence identity to SEQ ID NO: 11, at least 60% sequence identity to SEQ ID NO: 12, at least 60% sequence identity to SEQ ID NO: 13, at least 55% sequence identity to SEQ ID NO: 14, at least 50% sequence identity to SEQ ID NO: 15, at least 35% sequence identity to SEQ ID NO: 16, at least 55% sequence identity to SEQ ID NO: 17, at least 50% sequence identity to SEQ ID NO: 18, at least 40% sequence identity to SEQ ID NO: 19, at least 30% sequence identity to SEQ ID NO: 20, at least 55% sequence identity to SEQ ID NO: 21, at least 40% sequence identity to SEQ ID NO: 22, at least 40% sequence identity to SEQ ID NO: 23, at least 40% sequence identity to SEQ ID NO: 24, at least 30% sequence identity to SEQ ID NO: 25, at least 50% sequence identity to SEQ ID NO: 26, at least 50% sequence identity to SEQ ID NO: 27, at least 35% sequence identity to SEQ ID NO: 28, at least 45% sequence identity to SEQ ID NO: 29, at least 45% sequence identity to SEQ ID NO: 30, at least 40% sequence identity to SEQ ID NO: 31, at least 60% sequence identity to SEQ ID NO: 32, at least 45% sequence identity to SEQ ID NO: 33, at least 35% sequence identity to SEQ ID NO: 34, at least 50% sequence identity to SEQ ID NO: 35, at least 60% sequence identity to SEQ ID NO: 36, at least 40% sequence identity to SEQ ID NO: 37, at least 40% sequence identity to SEQ ID NO: 38, at least 50% sequence identity to SEQ ID NO: 39, at least 55% sequence identity to SEQ ID NO: 40, at least 40% sequence identity to SEQ ID NO: 41, at least 40% sequence identity to SEQ ID NO: 42, at least 40% sequence identity to SEQ ID NO: 43, at least 55% sequence identity to SEQ ID NO: 44, at least 35% sequence identity to SEQ ID NO: 45, at least 50% sequence identity to SEQ ID NO: 46, at least 40% sequence identity to SEQ ID NO: 47, at least 35% sequence identity to SEQ ID NO: 48, at least 55% sequence identity to SEQ ID NO: 49, at least 50% sequence identity to SEQ ID NO: 50, at least 55% sequence identity to SEQ ID NO: 51, at least 60% sequence identity to SEQ ID NO: 52, at least 30% sequence identity to SEQ ID NO: 53, at least 65% sequence identity to SEQ ID NO: 54, at least 40% sequence identity to SEQ ID NO: 55, at least 65% sequence identity to SEQ ID NO: 56, at least 35% sequence identity to SEQ ID NO: 57, at least 60% sequence identity to SEQ ID NO: 58, at least 70% sequence identity to SEQ ID NO: 59, at least 60% sequence identity to SEQ ID NO: 60, at least 40% sequence identity to SEQ ID NO: 61, at least 30% sequence identity to SEQ ID NO: 62, at least 35% sequence identity to SEQ ID NO: 63, at least 35% sequence identity to SEQ ID NO: 64, at least 45% sequence identity to SEQ ID NO: 65, at least 60% sequence identity to SEQ ID NO: 66, at least 60% sequence identity to SEQ ID NO: 67, at least 45% sequence identity to SEQ ID NO: 68, at least 35% sequence identity to SEQ ID NO: 69, at least 55% sequence identity to SEQ ID NO: 70, at least 45% sequence identity to SEQ ID NO: 71, at least 40% sequence identity to SEQ ID NO: 72, at least 65% sequence identity to SEQ ID NO: 73, at least 50% sequence identity to SEQ ID NO: 74, at least 85% sequence identity to SEQ ID NO: 75, at least 85% sequence identity to SEQ ID NO: 76, at least 35% sequence identity to SEQ ID NO: 77, at least 50% sequence identity to SEQ ID NO: 78, at least 50% sequence identity to

SEQ ID NO: 79, at least 45% sequence identity to SEQ ID NO: 80, at least 55% sequence identity to SEQ ID NO: 81, at least 40% sequence identity to SEQ ID NO: 82, at least 35% sequence identity to SEQ ID NO: 83, at least 45% sequence identity to SEQ ID NO: 84, at least 50% sequence identity to SEQ ID NO: 85, at least 60% sequence identity to SEQ ID NO: 86, at least 50% sequence identity to SEQ ID NO: 87, at least 50% sequence identity to SEQ ID NO: 88, at least 55% sequence identity to SEQ ID NO: 89, at least 50% sequence identity to SEQ ID NO: 90, at least 75% sequence identity to SEQ ID NO: 91, at least 55% sequence identity to SEQ ID NO: 92, at least 45% sequence identity to SEQ ID NO: 93, at least 50% sequence identity to SEQ ID NO: 94, at least 55% sequence identity to SEQ ID NO: 95, at least 55% sequence identity to SEQ ID NO: 96, at least 45% sequence identity to SEQ ID NO: 97, at least 45% sequence identity to SEQ ID NO: 98, at least 55% sequence identity to SEQ ID NO: 99, at least 55% sequence identity to SEQ ID NO: 100, at least 30% sequence identity to SEQ ID NO: 101, at least 35% sequence identity to SEQ ID NO: 102, at least 45% sequence identity to SEQ ID NO: 103, at least 55% sequence identity to SEQ ID NO: 104 and at least 50% sequence identity to SEQ ID NO: 105.

Preferably, the composition of the invention comprises a glycosyl hydrolase endogenous to *Alkalitalea saponilacus* or a variant thereof selected from the group consisting of glycosyl hydrolase having at least 35%, 45%, 55%, 65%, 75%, 80%, 85%, 90%, 95%, 97%, 98%, 100% sequence identity to SEQ ID NO: 1, at least 55%, 65%, 75%, 80%, 85%, 90%, 95%, 97%, 98%, 100% sequence identity to SEQ ID NO: 2, at least 65%, 75%, 80%, 85%, 90%, 95%, 97%, 98%, 100% sequence identity to SEQ ID NO: 3, at least 55%, 65%, 75%, 80%, 85%, 90%, 95%, 97%, 98%, 100% sequence identity to SEQ ID NO: 4, at least 60% sequence identity to SEQ ID NO: 5, at least 55%, 65%, 75%, 80%, 85%, 90%, 95%, 97%, 98%, 100% sequence identity to SEQ ID NO: 6, at least 55%, 65%, 75%, 80%, 85%, 90%, 95%, 97%, 98%, 100% sequence identity to SEQ ID NO: 7, at least 55%, 65%, 75%, 80%, 85%, 90%, 95%, 97%, 98%, 100% sequence identity to SEQ ID NO: 8, at least 40%, 45%, 55%, 65%, 75%, 80%, 85%, 90%, 95%, 97%, 98%, 100% sequence identity to SEQ ID NO: 9, at least 50%, 55%, 65%, 75%, 80%, 85%, 90%, 95%, 97%, 98%, 100% sequence identity to SEQ ID NO: 10, at least 60% sequence identity to SEQ ID NO: 11, at least 60%, 65%, 75%, 80%, 85%, 90%, 95%, 97%, 98%, 100% sequence identity to SEQ ID NO: 12, at least 65%, 75%, 80%, 85%, 90%, 95%, 97%, 98%, 100% sequence identity to SEQ ID NO: 13, at least 55%, 65%, 75%, 80%, 85%, 90%, 95%, 97%, 98%, 100% sequence identity to SEQ ID NO: 14, at least 55%, 65%, 75%, 80%, 85%, 90%, 95%, 97%, 98%, 100% sequence identity to SEQ ID NO: 15, at least 35%, 45%, 55%, 65%, 75%, 80%, 85%, 90%, 95%, 97%, 98%, 100% sequence identity to SEQ ID NO: 16, at least 55%, 65%, 75%, 80%, 85%, 90%, 95%, 97%, 98%, 100% sequence identity to SEQ ID NO: 17, at least 50%, 60%, 65%, 75%, 80%, 85%, 90%, 95%, 97%, 98%, 100% sequence identity to SEQ ID NO: 18, at least 50%, 60%, 65%, 75%, 80%, 85%, 90%, 95%, 97%, 98%, 100% sequence identity to SEQ ID NO: 19, at least 35%, 45%, 55%, 65%, 75%, 80%, 85%, 90%, 95%, 97%, 98%, 100% sequence identity to SEQ ID NO: 20, at least 65%, 75%, 80%, 85%, 90%, 95%, 97%, 98%, 100% sequence identity to SEQ ID NO: 21, at least 50%, 55%, 65%, 75%, 80%, 85%, 90%, 95%, 97%, 98%, 100% sequence identity to SEQ ID NO: 22, at least 50%, 55%, 65%, 75%, 80%, 85%, 90%, 95%, 97%, 98%, 100% sequence identity to SEQ ID NO: 23, at least 50%, 55%, 65%, 75%, 80%, 85%, 90%, 95%, 97%, 98%, 100% sequence identity to SEQ ID NO: 24, at least 40%, 50%, 55%, 65%, 75%, 80%, 85%, 90%, 95%, 97%, 98%, 100% sequence identity to SEQ ID NO: 25, at least 60%, 65%, 75%, 80%, 85%, 90%, 95%, 97%, 98%, 100% sequence identity to SEQ ID NO: 26, at least 60%, 65%, 75%, 80%, 85%, 90%, 95%, 97%, 98%, 100% sequence identity to SEQ ID NO: 27, at least 45%, 55%, 65%, 75%, 80%, 85%, 90%, 95%, 97%, 98%, 100% sequence identity to SEQ ID NO: 28, at least 55%, 65%, 75%, 80%, 85%, 90%, 95%, 97%, 98%, 100% sequence identity to SEQ ID NO: 29, at least 55%, 65%, 75%, 80%, 85%, 90%, 95%, 97%, 98%, 100% sequence identity to SEQ ID NO: 30, at least 50%, 55%, 65%, 75%, 80%, 85%, 90%, 95%, 97%, 98%, 100% sequence identity to SEQ ID NO: 31, at least 65%, 75%, 80%, 85%, 90%, 95%, 97%, 98%, 100% sequence identity to SEQ ID NO: 32, at least 45% sequence identity to SEQ ID NO: 33, at least 40%, 50%, 55%, 65%, 75%, 80%, 85%, 90%, 95%, 97%, 98%, 100% sequence identity to SEQ ID NO: 34, at least 55%, 65%, 75%, 80%, 85%, 90%, 95%, 97%, 98%, 100% sequence identity to SEQ ID NO: 35, at least 65%, 75%, 80%, 85%, 90%, 95%, 97%, 98%, 100% sequence identity to SEQ ID NO: 36, at least 45%, 50%, 55%, 65%, 75%, 80%, 85%, 90%, 95%, 97%, 98%, 100% sequence identity to SEQ ID NO: 37, at least 45%, 50%, 55%, 65%, 75%, 80%, 85%, 90%, 95%, 97%, 98%, 100% sequence identity to SEQ ID NO: 38, at least 50% sequence identity to SEQ ID NO: 39, at least 65%, 75%, 80%, 85%, 90%, 95%, 97%, 98%, 100% sequence identity to SEQ ID NO: 40, at least 50%, 55%, 65%, 75%, 80%, 85%, 90%, 95%, 97%, 98%, 100% sequence identity to SEQ ID NO: 41, at least 50%, 55%, 65%, 75%, 80%, 85%, 90%, 95%, 97%, 98%, 100% sequence identity to SEQ ID NO: 42, at least 50%, 55%, 65%, 75%, 80%, 85%, 90%, 95%, 97%, 98%, 100% sequence identity to SEQ ID NO: 43, at least 65%, 75%, 80%, 85%, 90%, 95%, 97%, 98%, 100% sequence identity to SEQ ID NO: 44, at least 45%, 50%, 55%, 65%, 75%, 80%, 85%, 90%, 95%, 97%, 98%, 100% sequence identity to SEQ ID NO: 45, at least 55%, 65%, 75%, 80%, 85%, 90%, 95%, 97%, 98%, 100% sequence identity to SEQ ID NO: 46, at least 50%, 55%, 65%, 75%, 80%, 85%, 90%, 95%, 97%, 98%, 100% sequence identity to SEQ ID NO: 47, at least 40%, 45%, 50%, 55%, 65%, 75%, 80%, 85%, 90%, 95%, 97%, 98%, 100% sequence identity to SEQ ID NO: 45, at least 55%, 65%, 75%, 80%, 85%, 90%, 95%, 97%, 98%, 100% sequence identity to SEQ ID NO: 48, at least 65%, 75%, 80%, 85%, 90%, 95%, 97%, 98%, 100% sequence identity to SEQ ID NO: 49, at least 55%, 65%, 75%, 80%, 85%, 90%, 95%, 97%, 98%, 100% sequence identity to SEQ ID NO: 50, at least 65%, 75%, 80%, 85%, 90%, 95%, 97%, 98%, 100% sequence identity to SEQ ID NO: 51, at least 60%, 75%, 80%, 85%, 90%, 95%, 97%, 98%, 100% sequence identity to SEQ ID NO: 52, at least 30% sequence identity to SEQ ID NO: 53, at least 65% sequence identity to

SEQ ID NO: 54, at least 50%, 75%, 80%, 85%, 90%, 95%, 97%, 98%, 100% sequence identity to SEQ ID NO: 55, at least 75%, 80%, 85%, 90%, 95%, 97%, 98%, 100% sequence identity to SEQ ID NO: 56, at least 45%, 55%, 65%, 75%, 80%, 85%, 90%, 95%, 97%, 98%, 100% sequence identity to SEQ ID NO: 57, at least 60% sequence identity to SEQ ID NO: 58, at least 70% sequence identity to SEQ ID NO: 59, at least 65%, 75%, 80%, 85%, 90%, 95%, 97%, 98%, 100% sequence identity to SEQ ID NO: 60, at least 50%, 65%, 75%, 80%, 85%, 90%, 95%, 97%, 98%, 100% sequence identity to SEQ ID NO: 61, at least 35%, 45%, 55%, 65%, 75%, 80%, 85%, 90%, 95%, 97%, 98%, 100% sequence identity to SEQ ID NO: 62, at least 40%, 45%, 55%, 65%, 75%, 80%, 85%, 90%, 95%, 97%, 98%, 100% sequence identity to SEQ ID NO: 63, at least 40%, 45%, 55%, 65%, 75%, 80%, 85%, 90%, 95%, 97%, 98%, 100% sequence identity to SEQ ID NO: 64, at least 55%, 65%, 75%, 80%, 85%, 90%, 95%, 97%, 98%, 100% sequence identity to SEQ ID NO: 65, at least 65%, 75%, 80%, 85%, 90%, 95%, 97%, 98%, 100% sequence identity to SEQ ID NO: 66, at least 65%, 75%, 80%, 85%, 90%, 95%, 97%, 98%, 100% sequence identity to SEQ ID NO: 67, at least 55%, 65%, 75%, 80%, 85%, 90%, 95%, 97%, 98%, 100% sequence identity to SEQ ID NO: 68, at least 45%, 55%, 65%, 75%, 80%, 85%, 90%, 95%, 97%, 98%, 100% sequence identity to SEQ ID NO: 69, at least 65%, 75%, 80%, 85%, 90%, 95%, 97%, 98%, 100% sequence identity to SEQ ID NO: 70, at least 55%, 65%, 75%, 80%, 85%, 90%, 95%, 97%, 98%, 100% sequence identity to SEQ ID NO: 71, at least 50%, 65%, 75%, 80%, 85%, 90%, 95%, 97%, 98%, 100% sequence identity to SEQ ID NO: 72, at least 75%, 80%, 85%, 90%, 95%, 97%, 98%, 100% sequence identity to SEQ ID NO: 73, at least 55%, 65%, 75%, 80%, 85%, 90%, 95%, 97%, 98%, 100% sequence identity to SEQ ID NO: 74, at least 95%, 98%, 100% sequence identity to SEQ ID NO: 75, at least 85% sequence identity to SEQ ID NO: 76, at least 35% sequence identity to SEQ ID NO: 77, at least 55%, 65%, 75%, 80%, 85%, 90%, 95%, 97%, 98%, 100% sequence identity to SEQ ID NO: 78, at least 55%, 65%, 75%, 80%, 85%, 90%, 95%, 97%, 98%, 100% sequence identity to SEQ ID NO: 79, at least 50%, 55%, 65%, 75%, 80%, 85%, 90%, 95%, 97%, 98%, 100% sequence identity to SEQ ID NO: 80, at least 60%, 65%, 75%, 80%, 85%, 90%, 95%, 97%, 98%, 100% sequence identity to SEQ ID NO: 81, at least 50%, 55%, 65%, 75%, 80%, 85%, 90%, 95%, 97%, 98%, 100% sequence identity to SEQ ID NO: 82, at least 45%, 50%, 55%, 65%, 75%, 80%, 85%, 90%, 95%, 97%, 98%, 100% sequence identity to SEQ ID NO: 83, at least 50%, 55%, 65%, 75%, 80%, 85%, 90%, 95%, 97%, 98%, 100% sequence identity to SEQ ID NO: 84, at least 55%, 65%, 75%, 80%, 85%, 90%, 95%, 97%, 98%, 100% sequence identity to SEQ ID NO: 85, at least 60% sequence identity to SEQ ID NO: 86, at least 55%, 65%, 75%, 80%, 85%, 90%, 95%, 97%, 98%, 100% sequence identity to SEQ ID NO: 87, at least 55%, 65%, 75%, 80%, 85%, 90%, 95%, 97%, 98%, 100% sequence identity to SEQ ID NO: 88, at least 60%, 65%, 75%, 80%, 85%, 90%, 95%, 97%, 98%, 100% sequence identity to SEQ ID NO: 89, at least 55%, 65%, 75%, 80%, 85%, 90%, 95%, 97%, 98%, 100% sequence identity to SEQ ID NO: 90, at least 75% sequence identity to SEQ ID NO: 91, at least 60%, 65%, 75%, 80%, 85%, 90%, 95%, 97%, 98%, 100% sequence identity to SEQ ID NO: 92, at least 55%, 65%, 75%, 80%, 85%, 90%, 95%, 97%, 98%, 100% sequence identity to SEQ ID NO: 93, at least 50% sequence identity to SEQ ID NO: 94, at least 55% sequence identity to SEQ ID NO: 95, at least 55% sequence identity to SEQ ID NO: 96, at least 55%, 65%, 75%, 80%, 85%, 90%, 95%, 97%, 98%, 100% sequence identity to SEQ ID NO: 97, at least 55%, 65%, 75%, 80%, 85%, 90%, 95%, 97%, 98%, 100% sequence identity to SEQ ID NO: 98, at least 60%, 65%, 75%, 80%, 85%, 90%, 95%, 97%, 98%, 100% sequence identity to SEQ ID NO: 99, at least 60%, 65%, 75%, 80%, 85%, 90%, 95%, 97%, 98%, 100% sequence identity to SEQ ID NO: 100, at least 40%, 50%, 55%, 65%, 75%, 80%, 85%, 90%, 95%, 97%, 98%, 100% sequence identity to SEQ ID NO: 101, at least 45%, 55%, 65%, 75%, 80%, 85%, 90%, 95%, 97%, 98%, 100% sequence identity to SEQ ID NO: 102, at least 55%, 65%, 75%, 80%, 85%, 90%, 95%, 97%, 98%, 100% sequence identity to SEQ ID NO: 103, at least 65%, 75%, 80%, 85%, 90%, 95%, 97%, 98%, 100% sequence identity to SEQ ID NO: 104 and at least 50%, 55%, 65%, 75%, 80%, 85%, 90%, 95%, 97%, 98%, 100% sequence identity to SEQ ID NO: 105.

Preferably, the composition of the invention comprises a glycosyl hydrolase endogenous to *Alkalitalea saponilacus* or a variant thereof selected from the group consisting of glycosyl hydrolases having at least 35%, preferably at least 45% sequence identity to SEQ ID NO: 1, at least 45%, preferably at least 55% sequence identity to SEQ ID NO: 2, at least 55%, preferably at least 65% sequence identity to SEQ ID NO: 3, at least 55%, preferably at least 65% sequence identity to SEQ ID NO: 49, at least 65%, preferably at least 75% sequence identity to SEQ ID NO: 54, at least 60%, preferably at least 70% sequence identity to SEQ ID NO: 58, at least 70%, preferably at least 80% sequence identity to SEQ ID NO: 59, at least 60%, preferably at least 70% sequence identity to SEQ ID NO: 60, at least 45%, preferably at least 55% sequence identity to SEQ ID NO: 71, at least 45%, preferably at least 55% sequence identity to SEQ ID NO: 93, at least 50%, preferably at least 60% sequence identity to SEQ ID NO: 94, at least 55%, preferably at least 65% sequence identity to SEQ ID NO: 95 and at least 30%, preferably at least 40% sequence identity to SEQ ID NO: 101.

Preferably, the composition of the invention comprises a glycosyl hydrolase endogenous to *Alkalitalea saponilacus* or a variant thereof selected from the group consisting of glycosyl hydrolases having at least 55%, preferably at least 85% sequence identity to SEQ ID NO: 1, at least 65%, preferably at least 85% sequence identity to SEQ ID NO: 2, at least 75%, preferably at least 90% sequence identity to SEQ ID NO: 3, at least 65%, preferably at least 85% sequence identity to SEQ ID NO: 49, at least 75%, preferably at least 90% sequence identity to SEQ ID NO: 54, at least 70%, preferably at least 90% sequence identity to SEQ ID NO: 58, at least 80%, preferably at least 95% sequence identity to SEQ ID NO: 59, at least 70%, preferably at least 90% sequence identity to SEQ ID NO: 60, at least 65%, preferably at least 85% sequence identity to SEQ ID NO: 71, at least 65%, preferably at least 85% sequence identity to SEQ ID NO: 93, at

least 70%, preferably at least 95% sequence identity to SEQ ID NO: 94, at least 75%, preferably at least 95% sequence identity to SEQ ID NO: 95, at least 50%, preferably at least 80% sequence identity to SEQ ID NO: 101 and a mixture thereof.

Preferably, the composition of the invention comprises a glycosyl hydrolase endogenous to *Alkalitalea saponilacus* or a variant thereof selected from the group consisting of glycosyl hydrolases having at least 95%, preferably at least 98%, more preferably 100% sequence identity to a sequence selected from the group consisting of SEQ ID NO: 1-3, 39, 49, 54, 58-60, 71, 93-95, 101 and a mixture thereof.

The glycosyl hydrolase endogenous to *Alkalitalea saponilacus* of SEQ ID NO: 1 provides good removal of cosmetic stains, such as foundation, from fabrics. The glycosyl hydrolase endogenous to *Alkalitalea saponilacus* of SEQ ID NO: 49 provides good removal of chocolate pudding from fabrics. The glycosyl hydrolase endogenous to *Alkalitalea saponilacus* of SEQ ID NO: 1 provides good removal of starch, such as oatmeal soil from dishware/tableware.

Preferred herein are:

i) a laundry composition comprising a glycosyl hydrolase endogenous to *Alkalitalea saponilacus* of SEQ ID NO: 1;
ii) a laundry composition comprising a glycosyl hydrolase endogenous to *Alkalitalea saponilacus* of SEQ ID NO: 49; and
iii) an automatic dishwashing composition comprising a glycosyl hydrolase endogenous to *Alkalitalea saponilacus* of SEQ ID NO: 1;

Polysaccharide lyase

Polysaccharide lyases (or eliminases) are a class of enzymes (EC 4.2.2.-) that act to cleave certain activated glycosidic linkages present in acidic polysaccharides. These enzymes act through an eliminase mechanism, rather than through hydrolysis, resulting in unsaturated oligosaccharide products.

Preferably, the composition of the invention comprises a polysaccharide lyase endogenous to *Alkalitalea saponilacus* or a variant thereof selected from the group consisting of polysaccharide lyase having at least 55% sequence identity to SEQ ID NO: 106, at least 50% sequence identity to SEQ ID NO: 107, at least 45% sequence identity to SEQ ID NO: 108, at least 55% sequence identity to SEQ ID NO: 109, at least 40% sequence identity to SEQ ID NO: 110 and at least 50% sequence identity to SEQ ID NO: 111.

Preferably, the composition of the invention comprises a polysaccharide lyase endogenous to *Alkalitalea saponilacus* or a variant thereof selected from the group consisting of polysaccharide lyase having at least 60%, 70%, 80%, 90%, 95%, 98%, 100% sequence identity to SEQ ID NO: 106, at least 60%, 70%, 80%, 90%, 95%, 98%, 100% sequence identity to SEQ ID NO: 107, at least 60%, 70%, 80%, 90%, 95%, 98%, 100% sequence identity to SEQ ID NO: 108, at least 60%, 70%, 80%, 90%, 95%, 98%, 100% sequence identity to SEQ ID NO: 109, at least 40%, 50%, 60%, 70%, 80%, 90%, 95%, 98%, 100% sequence identity to SEQ ID NO: 110 and at least 50%, 60%, 70%, 80%, 90%, 95%, 98%, 100% sequence identity to SEQ ID NO: 111.

Esterase / lipase

An esterase is a hydrolase enzyme that splits esters into an acid and an alcohol in hydrolysis. Lipase is a family of enzymes that catalyzes the hydrolysis of fats. Unlike esterases, which function in water, lipases "are activated only when adsorbed to an oil-water interface".

Preferably, the composition of the invention comprises a esterase / lipase endogenous to *Alkalitalea saponilacus* or a variant thereof selected from the group consisting of esterase / lipase having at least 50% sequence identity to SEQ ID NO: 112, at least 65% sequence identity to SEQ ID NO: 113, at least 45% sequence identity to SEQ ID NO: 114, at least 45% sequence identity to SEQ ID NO: 115, at least 40% sequence identity to SEQ ID NO: 116, at least 40% sequence identity to SEQ ID NO: 117, at least 70% sequence identity to SEQ ID NO: 118, at least 30% sequence identity to SEQ ID NO: 119, at least 30% sequence identity to SEQ ID NO: 120, at least 30% sequence identity to SEQ ID NO: 121, at least 50% sequence identity to SEQ ID NO: 122, at least 55% sequence identity to SEQ ID NO: 123 and at least 35% sequence identity to SEQ ID NO: 124.

Preferably, the composition of the invention comprises a esterase / lipase endogenous to *Alkalitalea saponilacus* or a variant thereof selected from the group consisting of esterase / lipase having at least 60%, 70%, 80%, 90%, 95%, 98%, 100% sequence identity to SEQ ID NO: 112, at least 75%, 85%, 90%, 95%, 98%, 100% sequence identity to SEQ ID NO: 113, at least 45%, 50%, 60%, 70%, 80%, 90%, 95%, 98%, 100% sequence identity to SEQ ID NO: 114, at least 50%, 60%, 70%, 80%, 90%, 95%, 98%, 100% sequence identity to SEQ ID NO: 115, at least 50%, 60%, 70%, 80%, 90%, 95%, 98%, 100% sequence identity to SEQ ID NO: 116, at least 40%, 50%, 60%, 70%, 80%, 90%, 95%, 98%, 100% sequence identity to SEQ ID NO: 117, at least 80%, 90%, 95%, 98%, 100% sequence identity to SEQ ID NO: 118, at least 40%, 50%, 60%, 70%, 80%, 90%, 95%, 98%, 100% sequence identity to SEQ ID NO: 119, at least 40%, 50%, 60%, 70%, 80%, 90%, 95%, 98%, 100% sequence identity to SEQ ID NO: 120, at least 40%, 50%, 60%, 70%, 80%, 90%, 95%, 98%, 100% sequence

identity to SEQ ID NO: 121, at least 60%, 70%, 80%, 90%, 95%, 98%, 100% sequence identity to SEQ ID NO: 122, at least 65%, 75%, 85%, 90%, 95%, 98%, 100% sequence identity to SEQ ID NO: 123 and at least 45%, 55%, 65%, 75%, 85%, 95%, 98%, 100% sequence identity to SEQ ID NO: 124.

Nuclease

A nuclease (also archaically known as nucleodepolymerase or polynucleotidase) is an enzyme capable of cleaving the phosphodiester bonds between nucleotides of nucleic acids.

Preferably, the composition of the invention comprises a nuclease endogenous to *Alkalitalea saponilacus* or a variant thereof selected from the group consisting of nuclease having at least 50% sequence identity to SEQ ID NO: 125, at least 50% sequence identity to SEQ ID NO: 126, at least 35% sequence identity to SEQ ID NO: 127, at least 40% sequence identity to SEQ ID NO: 128, at least 40% sequence identity to SEQ ID NO: 129, at least 60% sequence identity to SEQ ID NO: 130, at least 55% sequence identity to SEQ ID NO: 131, at least 30% sequence identity to SEQ ID NO: 132, at least 45% sequence identity to SEQ ID NO: 133, at least 50% sequence identity to SEQ ID NO: 134, at least 50% sequence identity to SEQ ID NO: 135, at least 30% sequence identity to SEQ ID NO: 136, at least 55% sequence identity to SEQ ID NO: 137, at least 40% sequence identity to SEQ ID NO: 138 and at least 40% sequence identity to SEQ ID NO: 139.

Preferably, the composition of the invention comprises a nuclease endogenous to *Alkalitalea saponilacus* or a variant thereof selected from the group consisting of nuclease having at least 60%, 70%, 80%, 90%, 95%, 98%, 100% sequence identity to SEQ ID NO: 125, at least 60%, 70%, 80%, 90%, 95%, 98%, 100% sequence identity to SEQ ID NO: 126, at least 45%, 55%, 65%, 75%, 85%, 95%, 98%, 100% sequence identity to SEQ ID NO: 127, at least 50%, 60%, 70%, 80%, 90%, 95%, 98%, 100% sequence identity to SEQ ID NO: 128, at least 50%, 60%, 70%, 80%, 90%, 95%, 98%, 100% sequence identity to SEQ ID NO: 129, at least 70%, 80%, 90%, 95%, 98%, 100% sequence identity to SEQ ID NO: 130, at least 65%, 75%, 85%, 95%, 98%, 100% sequence identity to SEQ ID NO: 131, at least 40%, 50%, 60%, 70%, 80%, 90%, 95%, 98%, 100% sequence identity to SEQ ID NO: 132, at least 55%, 65%, 75%, 85%, 95%, 98%, 100% sequence identity to SEQ ID NO: 133, at least 60%, 70%, 80%, 90%, 95%, 98%, 100% sequence identity to SEQ ID NO: 134, at least 60%, 70%, 80%, 90%, 95%, 98%, 100% sequence identity to SEQ ID NO: 135, at least 40%, 50%, 60%, 70%, 80%, 90%, 95%, 98%, 100% sequence identity to SEQ ID NO: 136, at least 65%, 75%, 85%, 95%, 98%, 100% sequence identity to SEQ ID NO: 137, at least 50%, 60%, 70%, 80%, 90%, 95%, 98%, 100% sequence identity to SEQ ID NO: 138 and at least 50%, 60%, 70%, 80%, 90%, 95%, 98%, 100% sequence identity to SEQ ID NO: 139

Protease / peptidase

A protease (also called a peptidase, proteinase, or proteolytic enzyme) is an enzyme that catalyzes proteolysis, breaking down proteins into smaller polypeptides or single amino acids.

Preferably, the composition of the invention comprises a protease / peptidase endogenous to *Alkalitalea saponilacus* or a variant thereof selected from the group consisting of protease / peptidase having at least 50% sequence identity to SEQ ID NO: 140, at least 50% sequence identity to SEQ ID NO: 141, at least 35% sequence identity to SEQ ID NO: 142, at least 40% sequence identity to SEQ ID NO: 143, at least 60% sequence identity to SEQ ID NO: 144, at least 35% sequence identity to SEQ ID NO: 145, at least 40% sequence identity to SEQ ID NO: 146, at least 30% sequence identity to SEQ ID NO: 147, at least 55% sequence identity to SEQ ID NO: 148, at least 55% sequence identity to SEQ ID NO: 149, at least 30% sequence identity to SEQ ID NO: 150, at least 30% sequence identity to SEQ ID NO: 151, at least 30% sequence identity to SEQ ID NO: 152, at least 30% sequence identity to SEQ ID NO: 153, at least 50% sequence identity to SEQ ID NO: 154, at least 50% sequence identity to SEQ ID NO: 155, at least 55% sequence identity to SEQ ID NO: 156, at least 40% sequence identity to SEQ ID NO: 157, at least 40% sequence identity to SEQ ID NO: 158, at least 50% sequence identity to SEQ ID NO: 159, at least 50% sequence identity to SEQ ID NO: 160, at least 45% sequence identity to SEQ ID NO: 161, at least 45% sequence identity to SEQ ID NO: 162, at least 65% sequence identity to SEQ ID NO: 163, at least 55% sequence identity to SEQ ID NO: 164, at least 30% sequence identity to SEQ ID NO: 165, at least 55% sequence identity to SEQ ID NO: 166, at least 45% sequence identity to SEQ ID NO: 167, at least 60% sequence identity to SEQ ID NO: 168, at least 55% sequence identity to SEQ ID NO: 169, at least 50% sequence identity to SEQ ID NO: 170, at least 45% sequence identity to SEQ ID NO: 171, at least 50% sequence identity to SEQ ID NO: 172, at least 50% sequence identity to SEQ ID NO: 173, at least 40% sequence identity to SEQ ID NO: 174, at least 45% sequence identity to SEQ ID NO: 175, at least 30% sequence identity to SEQ ID NO: 176, at least 50% sequence identity to SEQ ID NO: 177, at least 40% sequence identity to SEQ ID NO: 178, at least 45% sequence identity to SEQ ID NO: 179, at least 35% sequence identity to SEQ ID NO: 180, at least 55% sequence identity to SEQ ID NO: 181, at least 35% sequence identity to SEQ ID NO: 182, at least 50% sequence identity to SEQ ID NO: 183, at least 40% sequence identity to SEQ ID NO: 184, at least 30% sequence identity to SEQ ID NO: 185, at least 30% sequence identity to SEQ ID NO: 186, at least 55% sequence identity to SEQ ID NO: 187, at least 60% sequence identity to SEQ ID NO: 188 and at least 50% sequence identity to SEQ ID NO: 189 and at least 35% sequence identity to SEQ ID NO: 190.

Preferably, the composition of the invention comprises a protease / peptidase endogenous to *Alkalitalea saponilacus* or a variant thereof selected from the group consisting of protease / peptidase having at least 60%, 70%, 80%, 90%, 95%, 98%, 100% sequence identity to SEQ ID NO: 140, at least 60%, 70%, 80%, 90%, 95%, 98%, 100% sequence identity to SEQ ID NO: 141, at least 45%, 55%, 65%, 75%, 85%, 95%, 98%, 100% sequence identity to SEQ ID NO: 142, at least 50%, 60%, 70%, 80%, 90%, 95%, 98%, 100% sequence identity to SEQ ID NO: 143, at least 70%, 80%, 90%, 95%, 98%, 100% sequence identity to SEQ ID NO: 144, at least 45%, 55%, 65%, 75%, 85%, 95%, 98%, 100% sequence identity to SEQ ID NO: 145, at least 50%, 60%, 70%, 80%, 90%, 95%, 98%, 100% sequence identity to SEQ ID NO: 146, at least 40%, 50%, 60%, 70%, 80%, 90%, 95%, 98%, 100% sequence identity to SEQ ID NO: 147, at least 65%, 75%, 85%, 95%, 98%, 100% sequence identity to SEQ ID NO: 148, at least 65%, 75%, 85%, 95%, 98%, 100% sequence identity to SEQ ID NO: 149, at least 40%, 50%, 60%, 70%, 80%, 90%, 95%, 98%, 100% sequence identity to SEQ ID NO: 150, at least 40%, 50%, 60%, 70%, 80%, 90%, 95%, 98%, 100% sequence identity to SEQ ID NO: 151, at least 40%, 50%, 60%, 70%, 80%, 90%, 95%, 98%, 100% sequence identity to SEQ ID NO: 152, at least 40%, 50%, 60%, 70%, 80%, 90%, 95%, 98%, 100% sequence identity to SEQ ID NO: 153, at least 60%, 70%, 80%, 90%, 95%, 98%, 100% sequence identity to SEQ ID NO: 154, at least 60%, 70%, 80%, 90%, 95%, 98%, 100% sequence identity to SEQ ID NO: 155, at least 65%, 75%, 85%, 95%, 98%, 100% sequence identity to SEQ ID NO: 156, at least sequence identity to SEQ ID NO: 157, at least 50%, 60%, 70%, 80%, 90%, 95%, 98%, 100% sequence identity to SEQ ID NO: 158, at least 60%, 70%, 80%, 90%, 95%, 98%, 100% sequence identity to SEQ ID NO: 159, at least 50%, 60%, 70%, 80%, 90%, 95%, 98%, 100% sequence identity to SEQ ID NO: 160, at least 55%, 65%, 75%, 85%, 95%, 98%, 100% sequence identity to SEQ ID NO: 161, at least 55%, 65%, 75%, 85%, 95%, 98%, 100% sequence identity to SEQ ID NO: 162, at least 75%, 85%, 95%, 98%, 100% sequence identity to SEQ ID NO: 163, at least 65%, 75%, 85%, 95%, 98%, 100% sequence identity to SEQ ID NO: 164, at least 45%, 75%, 85%, 95%, 98%, 100% sequence identity to SEQ ID NO: 165, at least 65%, 75%, 85%, 95%, 98%, 100% sequence identity to SEQ ID NO: 166, at least 55%, 65%, 75%, 85%, 95%, 98%, 100% sequence identity to SEQ ID NO: 167, at least 70%, 80%, 90%, 95%, 98%, 100% sequence identity to SEQ ID NO: 168, at least 65%, 75%, 85%, 95%, 98%, 100% sequence identity to SEQ ID NO: 169, at least 60%, 70%, 80%, 90%, 95%, 98%, 100% sequence identity to SEQ ID NO: 170, at least 55%, 65%, 75%, 85%, 95%, 98%, 100% sequence identity to SEQ ID NO: 171, at least 60%, 70%, 80%, 90%, 95%, 98%, 100% sequence identity to SEQ ID NO: 172, at least 60%, 70%, 80%, 90%, 95%, 98%, 100% sequence identity to SEQ ID NO: 173, at least 50%, 60%, 70%, 80%, 90%, 95%, 98%, 100% sequence identity to SEQ ID NO: 174, at least 55%, 65%, 75%, 85%, 95%, 98%, 100% sequence identity to SEQ ID NO: 175, at least 40%, 50%, 60%, 70%, 80%, 90%, 95%, 98%, 100% sequence identity to SEQ ID NO: 176, at least 60%, 70%, 80%, 90%, 95%, 98%, 100% sequence identity to SEQ ID NO: 177, at least 50%, 60%, 70%, 80%, 90%, 95%, 98%, 100% sequence identity to SEQ ID NO: 178, at least 55%, 65%, 75%, 85%, 95%, 98%, 100% sequence identity to SEQ ID NO: 179, at least 45%, 55%, 65%, 75%, 85%, 95%, 98%, 100% sequence identity to SEQ ID NO: 180, at least 65%, 75%, 85%, 95%, 98%, 100% sequence identity to SEQ ID NO: 181, at least 45%, 55%, 65%, 75%, 85%, 95%, 98%, 100% sequence identity to SEQ ID NO: 182, at least 60%, 70%, 80%, 90%, 95%, 98%, 100% sequence identity to SEQ ID NO: 183, at least 50%, 60%, 70%, 80%, 90%, 95%, 98%, 100% sequence identity to SEQ ID NO: 184, at least 40%, 50%, 60%, 70%, 80%, 90%, 95%, 98%, 100% sequence identity to SEQ ID NO: 185, at least 40%, 50%, 60%, 70%, 80%, 90%, 95%, 98%, 100% sequence identity to SEQ ID NO: 186, at least 65%, 75%, 85%, 95%, 98%, 100% sequence identity to SEQ ID NO: 187, at least 70%, 80%, 90%, 95%, 98%, 100% sequence identity to SEQ ID NO: 188, at least 60%, 70%, 80%, 90%, 95%, 98%, 100% sequence identity to SEQ ID NO: 189 and at least 45%, 55%, 65%, 75%, 85%, 95%, 98%, 100% sequence identity to SEQ ID NO: 190.

Preferably the enzyme of the invention is present in the aqueous wash liquor in an amount of from 0.01ppm to 1000 ppm of the enzyme, or from 0.05 or from 0.1ppm to 750 or 500ppm.

Preferably the enzyme of the invention is present in the cleaning composition in an amount from 0.00005 to 5 wt.% based on active protein in the composition, or 0.001 to 1 wt.%, or from 0.005 to 0.5 wt.% or from 0.01 to 0.25 wt.% based on weight of the composition.

The composition comprises optional cleaning adjunct. Typically, the cleaning adjunct will be present in the composition in an amount from 1 to 98.9 wt.%, more typically from 5 to 90 wt.% cleaning adjunct. Suitable cleaning adjuncts comprise: colorants, chelating agents, dye transfer agents, deposition aids, dispersants, enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide;

dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof. The composition may be such that the cleaning adjunct comprises one or more selected from the group consisting of (i) perfume microcapsule; (ii) fabric hueing agent; (iii) protease; (iv) amphiphilic cleaning polymer; (v) amylase, or (vi) mixtures thereof.

Anionic Surfactant

The composition of the invention can provide good soil breakdown, however the removal of the products of the breakdown of the substrates and soils containing them is improved by the presence of anionic surfactant. Therefore, the laundry composition preferably comprises from 1 to 60 wt% an anionic surfactant. Preferably the weight ratio of anionic surfactant to active enzyme protein of the invention is at least 500:1, preferably at least 1000:1 or at least 1500:1 or at least 2000:1, preferably being no greater than 500000:1, preferably no greater than 400000: 1, or no greater than 200000: 1 or up to 150000: 1 or 100000: 1, or 50000: 1 or 10000: 1.

Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl-or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The invention also provides a cleaning composition comprising: from 0.00005 to 5 wt% (active enzyme protein) of the enzyme of the invention and a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ™, Lutensol™ and Pluronic™ from BASF, Dehypon™ series from Cognis and Genapol™ series from Clariant.

The cleaning composition preferably comprises from 0.5wt% to about 40wt% of a non-ionic surfactant, preferably 1 to 30 wt.% of the composition non-ionic surfactant.

The cleaning composition preferably comprises one or more additional enzymes selected from the group consisting of aminopeptidase, amylase, carbohydrase, carboxypeptidase, catalase, cellulase, chitinase, cutinase,

cyclodextrin glycosyltransferase, esterase, alpha-galactosidase, beta-galactosidase, glucoamylase, alpha-glucosidase, beta-glucosidase, haloperoxidase, hexosaminidase, invertase, laccase, lipase, mannanase, mannosidase, nuclease, oxidase, pectinolytic enzyme, peptidoglutaminase, peroxidase, phytase, polyphenoloxidase, proteolytic enzyme, transglutaminase, xylanase, xanthan lyase, xanthanase, endo-β-1,3-glucanase and mixtures thereof. Preferably the cleaning composition comprises additional enzyme selected from nuclease, such as DNase or RNase, mannanase, xanthan lyase, xanthanase, amylase and mixtures thereof.

Preferably the composition comprises additional enzymes selected from xanthan lyase, xanthanase, mannanase and mixtures thereof. Mannanase is particularly preferred.

The additional enzyme(s) may be produced, for example, by a microorganism belonging to the genus *Aspergillus*, *e.g., Aspergillus aculeatus, Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis, Fusarium crookwellense*, *Fusarium culmorum, Fusarium graminearum, Fusarium graminum, Fusarium heterosporum, Fusarium negundi, Fusarium oxysporum*, *Fusarium reticulatum, Fusarium roseum, Fusarium sambucinum, Fusarium sarcochroum, Fusarium sulphureum, Fusarium toruloseum, Fusarium trichothecioides,* or *Fusarium venenatum; Humicola, e.g., Humicola insolens* or *Humicola lanuginosa;* or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease and and one or more amylolytic enzymes, e.g., an alpha-amylase, glucoamylase, maltogenic amylase. In the case of laundry compositions, the composition can optionally comprise lipase or mixture of more than one lipase, a peroxidase or mixture of more than one peroxidase, and/or a cellulase or mixture thereof.

In general, the properties of the chosen enzyme(s) should be compatible with the selected composition, (i.e., pH-optimum, compatibility with other enzymatic and non-enzymatic ingredients, etc.), and the enzyme(s) should be present in effective amounts. Preferably, the composition of the invention comprises at least 0.01 mg, preferably from about 0.05 to about 10, more preferably from about 0.1 to about 6, especially from about 0.2 to about 5 mg of active further enzyme/ g of composition.

Proteases: The composition of the invention preferably comprises a protease. A mixture of two or more proteases can contribute to an enhanced cleaning across a broader temperature, cycle duration, and/or substrate range. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
Subtilisins (EC 3.4.21.62), especially those derived from *Bacillus,* such as *Bacillus sp., B. lentus, B. alkalophilus, B. subtilis, B. amyloliquefaciens, B. pumilus, B. gibsonii,* and *B. akibai* described in WO2004067737, WO2015091989, WO2015091990, WO2015024739, WO2015143360, US 6,312,936 B1, US 5,679,630, US 4,760,025, DE102006022216A1, DE102006022224A1, WO2015089447, WO2015089441, WO2016066756, WO2016066757, WO2016069557, WO2016069563, WO2016069569 and WO2016174234. Specifically, mutations S9R, A15T, V66A, A188P, V199I, Q239R, N255D (savinase numbering system).

Trypsin-type or chymotrypsin-type proteases, such as trypsin (e.g., of porcine or bovine origin), including the Fusarium protease described in WO 89/06270 and the chymotrypsin proteases derived from *Cellumonas* described in WO 05/052161 and WO 05/052146.

Metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* described in WO2014194032, WO2014194054 and WO2014194117; from *Kribella alluminosa* described in WO2015193488; and from *Streptomyces* and *Lysobacter* described in WO2016075078.

Protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Especially preferred proteases for the cleaning composition of the invention are polypeptides having at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).

(i) G118V + S128L + P129Q + S130A

(ii) S101M + G118V + S128L + P129Q + S130A

(iii) N76D + N87R + G118R + S128L + P129Q + S130A + S188D + N248R

(iv) N76D + N87R + G118R + S128L + P129Q + S130A + S188D + V244R

(v) N76D + N87R + G118R + S128L + P129Q + S130A

(vi) V68A + N87S + S101G + V104N

(vii) S99AD

(viii) S9R+A15T+V68A+N218D+Q245R

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase®, Coronase®, Blaze®, Blaze Ultra® and Esperase® by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (Bacillus alkalophilus subtilisin with mutations A230V + S256G + S259N) from Kao.

Especially preferred for use herein are commercial proteases selected from the group consisting of Properase®, Blaze®, Ultimase®, Everlase®, Savinase®, Excellase®, Blaze Ultra®, BLAP and BLAP variants.

Preferred levels of protease in the product of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces),* e.g., from *H. lanuginosa (T. lanuginosus)* or from *H. insolens,* a *Pseudomonas lipase,* e.g., from *P. alcaligenes* or *P. pseudoalcaligenes, P. cepacia P. stutzeri, P. fluorescens, Pseudomonas* sp. strain SD 705, *P. wisconsinensis* , a *Bacillus* lipase, e.g., from *B. subtilis* (Dartois et al. (1993), Biochemica et Biophysica Acta, 1131, 253-360), *B. stearothermophilus* or *B. pumilus .*

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot O59952 (derived from *Thermomyces lanuginosus (Humicola lanuginosa)).* Preferred lipases include those sold under the tradenames Lipex®, Lipolex® and Lipoclean®.

Other suitable lipases include: Liprl 139, e.g., as described in WO2013/171241; TfuLip2, e.g., as described in WO2011/084412 and WO2013/033318; Pseudomonas stutzeri lipase, e.g., as described in WO2018228880; *Microbulbifer thermotolerans* lipase, e.g., as described in WO2018228881; *Sulfobacillus acidocaldarius* lipase, e.g., as described in EP3299457; LIP062 lipase e.g., as described in WO2018209026; PinLip lipase e.g., as described in WO2017036901 and Absidia sp. lipase e.g., as described in WO2017005798.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium*, e.g., the fungal cellulases produced from *Humicola insolens, Myceliophthora thermophila* and *Fusarium oxysporum.* disclosed in US 4,435,307 , US 5,648,263 , US 5,691,178 , US 5,776,757 and US 5,691,178 .

In one aspect, preferred enzymes include microbial-derived endoglucanases exhibiting endo-beta-1,4-glucanase activity (E.C. 3.2.1.4), preferably selected from the group comprising:

(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 24 6, 237, 224 and 221 of the mature polypeptide of SEQ ID NO: 2, and the variant has c ellulase activity.;
(b) a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean® and Whitezyme® (Novozymes A/S, Bagsvaerd, Denmark). Examples include Celluclean® 5000L, Celluclean® Classic 400L, Celluclean® Classic 700T, Celluclean® 4500T, Whitezyme® 1.5T, Whitezyme® 2.0L.

Other commercially available cellulases include Celluzyme®, Carezyme®, Carezyme® Premium (Novozymes A/S), Clazinase®, Puradax HA®, Revitalenz® 1000, Revitalenz® 2000 (Genencor International Inc.), KAC-500(B)® (Kao Corporation), Biotouch® FCL, Biotouch® DCL, Biotouch® DCC, Biotouch® NCD, Biotouch® FCC, Biotouch® FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.
(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, I206, M208, D209, E212, V213, V214, N214, L217, R218, N219, V222, T225, T227, G229, I235, K242, Y243, S244, F245, T246, I250, S255, A256, H286, V291, T316, V317, V318, N417, T418, A419, H420, P421, I428, M429, F440, R443, N444, K445, Q448, S451, A465, N470, S472.
(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.
(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.
(f) variants exhibiting at least 89% identity with SEQ ID NO:1 in WO2016091688, especially those comprising deletions at positions H183+G184 and additionally one or more mutations at positions 405, 421, 422 and/or 428.
(g) variants described in WO2014099523, especially those exhibiting at least 60% amino acid sequence identity with the "PcuAmyl α-amylase" from *Paenibacillus curdlanolyticus* YK9 (SEQ ID NO:3 in WO2014099523).

(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178*, G179*, T38N, N88H, N126Y, T129I, N134M, F153W, L171R, T180D, E187P, I203Y, G476K, G477E, Y303D.

(i) variants exhibiting at least 85% identity with AmyE from *Bacillus subtilis* (SEQ ID NO:1 in WO2009149271).

(j) variants exhibiting at least 90% identity with the wild-type amylase from Bacillus sp. KSM-K38 with accession number AB051102.

(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H*, N54S, V56T, K72R, G109A, F113Q, R116Q, W167F, Q172G, A174S, G184T, N195F, V206L, K391A, P473R, G476K.

(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL®, ATLANTIC®, ACHIEVE ALPHA®, AMPLIFY® PRIME, INTENSA® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS®, POWERASE®, PREFERENZ S® series (including PREFERENZ S1000® and PREFERENZ S2000® and PURASTAR OXAM® (DuPont., Palo Alto, California) and KAM® (Kao, 14-10 Nihonbashi Kayabacho, 1-chome, Chuo-ku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from *C. cinereus,* and variants thereof as those described in WO 93/24618, WO 95/10602, and WO 98/15257.

Commercially available peroxidases include GUARDZYME® (Novozymes A/S).

Pectate lyase: Suitable pectate lyases include those sold under the tradenames Pectawash®, Pectaway®, X-Pect®, (all Novozymes A/S, Bagsvaerd, Denmark) Preferenz® F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway® (Novozymes A/S) such as Mannaway® 200L and Mannaway Evity 4.0T Other commercially available mannanases include Effectenz® M1000, Mannastar® 375, Preferenz M100 and Purabrite® (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Nucleases: Preferably the composition comprises a nuclease enzyme such as a RNase or DNase or mixtures thereof. The nuclease enzyme is an enzyme capable of cleaving the phosphodiester bonds between the nucleotide subunits of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic

activity. Thus, it includes truncated, but functional versions, of the enzyme and/or variants and/or derivatives and/or homologues whose functionality is maintained.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof.

DNase: Suitable DNases include wild-types and variants of DNases defined by SEQ ID NOS: 1, 2, 3, 4, 5, 6, 7, 8 and 9 in WO2017162836 (Novozymes), and SEQ ID NOS: 1, 2, 3, 4, 5, 6, 7, 8, 9, 10, 11, 12, 13, 14, 15, 16, 17, 18, 19, 20, 21, 22, 23, 24, 25, 28, 29, 30, 31, 32, 33, 34, 35, 36, 37, 38 39, 40, 41, 42, 43, 44, 45, 46, 47, 48, 49 50, 51, 52, 53, and 54 in WO2018108865, and variants of the Bacillus cibi DNase including those described in WO2018011277 (Novozymes), incorporated herein by reference. Preferred DNases are as claimed in EP3476935A.

RNase: suitable RNases include wild-types and variants of DNases defined by SEQ ID NOS: 3, 6, 9, 12, 15, 57, 58, 59, 60, 61, 62, 63, 64, 65, 66, 67, 72 and 73 in WO2018178061 (Novozymes), and SEQ ID NOS: 86, 87, 88, 89, 90, 91, 92, 93, 94, 95, 96, 97, 98, 99, 100, 101, 102, 103 and 104 in WO2020074499 (Novozymes), incorporated herein by reference.

Hexosaminidase: The composition preferably additionally comprises one or more hexosaminidase enzymes. The term hexosaminidase includes "dispersin" and the abbreviation "Dsp", which means a polypeptide having hexosaminidase activity. Suitable enzymes are found in EC 3.2.1.- that catalyze the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 are preferred, especially the variants with improved thermostability disclosed in that publication.

Particularly preferred hexosaminidase enzymes are hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 8; and hexosaminidase enzymes having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 9 disclosed in WO2022/094164; and mixtures thereof.

Alpha-1, 4-polygalactosaminidase: The composition preferably comprises one or more alpha-1, 4-polygalactosaminidase enzymes (EC 3.2.1.109). The term alpha-1, 4-polygalactosaminidase includes "Pel-ase", Which means a polypeptide having activity against alpha-1,4-polygalactosamine residues, that can be further partially N-acetylated. Pel-ase activity may be determined by activity towards poly-galactosamine substrate according to assay described in WO2019228448. Suitable Pel-ases include polypeptides comprising a GH114 domain having hydrolytic activity towards poly-galactosamine, including those disclosed in WO2018102478, WO2019228448, WO2018185181, WO2020070011, WO2018185267, WO2020070209 or WO2020008043; and mixtures thereof.

The composition preferably comprises one or more enzymes having glycoside hydrolase activity and selected from glycoside hydrolase family GH 39, termed Psl-ases. Wherein the glycoside hydrolase comprises PslGh comprising a GH39 glycosyl hydrolase domain. Suitable Psl-ases include those disclosed in WO 2018102479, WO2018185280, WO2020008043; and mixtures thereof.

The composition preferably comprises one or more alginate lyase enzymes. The term alginate lyase includes polypeptides wherein the alginate lyase enzyme has activity towards poly(beta-D-mannuronate) (polyM activity) and/or activity towards poly(alpha-L-guluronate) (polyG activity) substrates. Alginate lyase activity may be determined according to assay described in WO2022094588. Suitable alginate lyases include those disclosed in WO2022094588 and WO2022094163.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

Galactanase: Preferably the composition comprises a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the detergent composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 or EP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint® Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenyl-methane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites,

useable in laundry preferably has the formula $Na_{12}(AlO_2)_{12}(SiO_2)_{12}$-$27H_2O$ and the particle size is usually between 1-10 μm for zeolite A and 0.7-2 um for zeolite MAP. Other builders are Sodium metasilicate ($Na_2SiO_3 \cdot nH_2O$ or $Na_2Si_2O_5 \cdot n\, H_2O$) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; antibacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.

Amphiphilic cleaning polymers: Preferably, the amphiphilic cleanimg polymer is a compound having the following general structure: $bis((C_2H_5O)(C_2H_4O)n)(CH_3)$-$N^+$-$C_xH_{2x}$-$N^+$-$(CH_3)$-$bis((C_2H_5O)(C_2H_4O)n)$, wherein n = from 20 to 30, and x = from 3 to 8, or sulphated or sulphonated variants thereof.

Amphiphilic alkoxylated grease cleaning polymers suitable for use in the composition of the invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV):

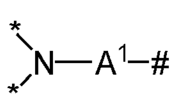
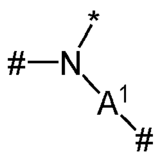
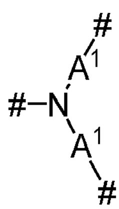
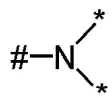

(I) (II) (III) (IV)

wherein # in each case denotes one-half of a bond between a nitrogen atom and the free binding position of a group $A^1$ of

two adjacent repeating units of formulae (I), (II), (III) or (IV); * in each case denotes one-half of a bond to one of the alkoxylate groups; and $A^1$ is independently selected from linear or branched $C_2$-$C_6$-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b),

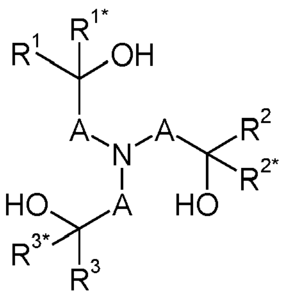

(I.a)

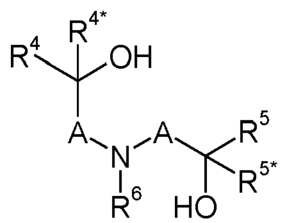

(I.b)

wherein A are independently selected from $C_1$-$C_6$-alkylene; $R^1$, $R^{1*}$, $R^2$, $R^{2*}$, $R^3$, $R^{3*}$, $R^4$, $R^{4*}$, $R^5$ and $R^{5*}$ are independently selected from hydrogen, alkyl, cycloalkyl or aryl, wherein the last three mentioned radicals may be optionally substituted; and $R^6$ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V)

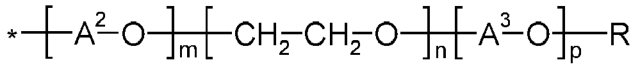

(V)

wherein * in each case denotes one-half of a bond to the nitrogen atom of the repeating unit of formula (I), (II) or (IV); $A^2$ is in each case independently selected from 1,2-propylene, 1,2-butylene and 1,2-isobutylene; $A^3$ is 1,2-propylene; R is in each case independently selected from hydrogen and $C_1$-$C_4$-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):

(I) $-[(OCHR^1-CHR^Z)_a-O-OC-Ar-CO-]_d$

(II) $-[(OCHR^3-CHR^4)_b-O-OC-sAr-CO-]_e$

(III) $-[(OCHR^5-CHR^6)_c-OR^7]_f$

wherein:

- a, b and c are from 1 to 200;
- d, e and f are from 1 to 50;
- Ar is a 1,4-substituted phenylene;
- sAr is 1,3-substituted phenylene substituted in position 5 with $SO_3Me$;
- Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
- $R^1$, $R^2$, $R^3$, $R^4$, $R^5$ and $R^6$ are independently selected from H or $C_1$-$C_{18}$ n- or iso-alkyl; and

$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N-diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g., fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or perfumes.

METHODS

The present invention also provides a method for treating a substrate, preferably a method for cleaning a substrate. The substrate can be a soft surface, such as a fabric or a hard surface, such as cookware and/or tableware. The method comprises a contacting step, contacting a surface preferably with an aqueous wash liquor comprising the enzyme of the invention, preferably in an amount from 0.01ppm to 10ppm, preferably from 0.1ppm to 1ppm; and preferably additionally a surfactant, preferably in an amount from 0.05 to 50g/l, more preferably from 0.2g/l to 5g/l or 0.5g/l to 3g/l.

The aqueous wash liquor may be formed by adding a composition as described above to water, for example in a washing machine, dishwasher or hand washing process. The surface may be optionally subsequently washed, and/or rinsed and/or dried.

The enzyme of the invention may be present in the wash liquor in an amount corresponding to 0.001-100 mg of active enzyme protein per litre of wash liquor, preferably 0.005-5 mg of active enzyme protein per litre of wash liquor, more preferably 0.01-1 mg of enzyme protein per litre of wash liquor and in particular 0.1-1 mg of enzyme protein per litre of wash.

In the contacting step, or in a subsequent step, it may be preferred to use mechanical agitation to promote cleaning and removal of the broken-down soil by-products from the surface. The wash liquor preferably has a pH of from about 7 or 8 to about 11.5. The composition may typically be employed at concentrations of from about 500 ppm to about 15,000 ppm in solution to form the wash liquor. The wash liquor preferably has a temperature from about 5°C to about 40°C, or preferably from 10 to 35°C or 30°C or 25°C. When the surface is a fabric, the water to fabric ratio is typically from about 1:1 to about 30:1.

Method for production of *Alkalitalea saponilicus* enzymes

The following general method was used for the recombinant cloning and expression of *Alkalitalea saponilicus* genes of interest and the purification of the encoded enzymes from E. *coli.*

Plasmid constructs encoding enzymes lacking their signal peptides were amplified by PCR using appropriate primers and a *Alkalitalea saponilacus* (DSM 24412) bacterial glycerol stock, as a template. The amplified PCR products were cloned into pET28a using NheI and XhoI restriction sites. The genes were then expressed in E. coli BL21(DE3) cells, transformed with the recombinant plasmids. The transformed E. *coli* strains were cultured in Luria-Bertani broth (LB) Miller supplemented with 50 μg/ml of kanamycin. Cultured cells were grown at 37°C to mid-log phase, induced with 0.5 mM isopropyl β-D-1-thiogalactopyranoside then grown at 16°C 100 rpm overnight. Cells were pelleted by centrifugation at 4000 x g for 10 min at 4°C and resuspended in 20 mM Tris-HCl buffer, 300 mM NaCl, pH 8.0. The resuspended pellets were disrupted by sonication and the cell-free extract was recovered by centrifugation at 24 000 x g for 30 min, 4°C. Recombinant proteins were purified from the cell-free extract using immobilised metal affinity chromatography using Talon®, a cobalt-based matrix, using a stepwise elution with imidazole. Purified recombinant proteins, as judged by SDS-PAGE, were buffer exchanged to remove imidazole using 10kDa MWCO protein concentrators. Protein concentrations were determined by measuring $A^{280}$ and converting to mg/mL using calculated extinction coefficients.

## EXAMPLES

### Example 1

The activity of *Alkalitalea saponilacus* endo-β-1,3-glucanase (SEQ ID NO: 1) and a laminarinase outside the scope of the invention (CZ08612, batch YL101, from NZYTech, Lisbon, Portugal) were measured towards β-1,3 glucans at different pHs (8, 9 and 10.5). Activity against β-1,3 glucans was measured using azo-curdlan substrate (Product code: T-CUR-200T, Megazyme).

One tablet of T-CUR-200T dissolved in a buffer containing sodium bicarbonate and sodium chloride, was adjusted to desired pH with 50% 12.5N sodium hydroxide (VWR) and incubated with 0.5 ppm of each laminarinase of interest at 40°C for 30 min, in a 96 well plate.

The activity towards each of the substrates at different pH conditions is given as the absorbance at 590 nm in a spectrophotometer plate reader vs nil enzyme sample when the enzyme was put into contact with the substrate. These values are then used to evaluate activity towards β-1,3 glucans. An enzyme having activity against β-1,3 glucans preferably provides an absorbance delta to nil enzyme of at least 1.5 absorbance units, more preferably at least 2.5 absorbance units and more preferably at least 3.5 absorbance units.

| | pH 8 | Absorbance (n=3) | SE (n=3) | p-value vs A (95%) | p-value vs B (95%) |
|---|---|---|---|---|---|
| A | Nil | 0.039 | 0.001 | | |
| B | GH16 (CZ08612, NZYtech) | 3.741 | 0.057 | 0002 | |
| C | *Alkalitalea saponilacus* endo-β-1,3-Glucanase (SEQ ID NO 1) | 3.650 | 0.047 | 0.0002 | 0.280 |

| Leg | pH 9 | Absorbance (n=3) | SE (n=3) | p-value vs A (95%) | p-value vs B (95%) |
|---|---|---|---|---|---|
| A | Nil | 0.019 | 0.009 | | |
| B | GH16 (CZ08612, NZYtech) | 3.700 | 0.143 | 3015 | |
| C | *Alkalitalea saponilacus* endo-β-1,3-Glucanase (SEQ ID NO 1) | 3.746 | 0.119 | 0.0009 | 0.809 |

| | pH 10.5 | Absorbance (n=3) | SE (n=3) | p-value vs A (95%) | p-value vs B (95%) |
|---|---|---|---|---|---|
| A | Nil | 0.012 | 0.009 | | |
| B | GH16 (CZ08612, NZYtech) | 1.596 | 0.282 | 0.030 | |

(continued)

| | pH 10.5 | Absorbance (n=3) | SE (n=3) | p-value vs A (95%) | p-value vs B (95%) |
|---|---|---|---|---|---|
| C | *Alkalitalea saponilacus* endo-β-1,3-Glucanase (SEQ ID NO 1) | 3.664 | 0.098 | 0.001 | 0.011 |

As it can be seen from the data above, *Alkalitalea saponilacus* endo-β-1,3-glucanase (SEQ ID NO: 1) shows high activity against β-1,3 glucans at pH 8, 9 and 10.5. Importantly, the activity of this endo-β-1,3-glucanase according to the invention (*Alkalitalea saponilacus* endo-β-1,3-glucanase (SEQ ID NO: 1)) is significantly higher than an enzyme outside the scope of the invention (GH16, CZ08612, NZYtech) at pH 10.5.

Example 2

The removal of foundation make-up stains from cotton fabrics was evaluated using a powder laundry detergent comprising an endo-β-1,3-glucanase according to the invention (SEQ ID NO: 1) and a powder detergent comprising an endo-β-1,3-glucanase outside the scope of the invention (CZ08612, batch YL101, from NZYTech, Lisbon, Portugal).

Stains were prepared on 5cm x 5cm swatches of knitted cotton supplied by Warwick Equest Ltd, Consett, U.K. Makeup (Living Nature foundation, Pure Taupe 30ml, batch 220129). The stains were applied by sponge on each swatch using a stencil with 2.5cm diameter circular holes. The stained swatches were left to line dry for 24 hours before use, and the colour of stains and white background fabric were image analysed for CIELab values (DigiEye, VeriVide, Leicester, U.K.) before testing.

Stain removal testing was conducted using a Tergotometer to simulate the washing of fabrics in a washing machine. Pots were filled with 1L of water (20 grains per US gallon) heated to 35°C, and 3.75g/L Tide Heavy Duty Granulate (prepared February 2023) was added to each pot. Two stain swatches were added to each pot, together with 10g of SBL2004 soil sheets cut into 5cm x 5cm swatches (WFK-Testgewebe GmbH, product code 10996) and sufficient 5cm x 5cm swatches of clean white knitted cotton ballast (Warwick Equest Ltd) to reach a total fabric load of 60g. The addition of 0.5mg active endo-β-1,3-glucanese enzyme (CZ08612, batch YL101, from NZYTech, Lisbon, Portugal) and 0.5mg active endo-β-1,3-glucanase (*Alkalitalea saponilacus,* SEQ ID NO: 1) were added via a pipette to each respective wash pot, resulting in a wash concentration of 0.5ppm active enzyme. The agitation was then initiated at 300rpm for 40 minutes and the temperature was maintained at 35°C for the duration of the test. After 40 minutes, the wash water was drained, and the fabrics rinsed twice for five minutes in 1L 15°C water (20 grains per US gallon) at 208rpm, before placing the washed stain swatches flat on a rack to dry under ambient conditions.

This process was repeated three more times, resulting in a total of eight washed stains per treatment, i.e., four external replicates, each comprising two stain swatches. The washed stains were analysed for CIELab as before, and Stain Removal Index (SRI) calculated using the following equation:

$$SRI = \left(\frac{\Delta E_{pre-wash} - \Delta E_{post-wash}}{\Delta E_{pre-wash}}\right) x\ 100$$

Where

$$\Delta E_{pre-wash} = \sqrt{[(L_{Clean} - L_{Stained})^2 + (a_{Clean} - a_{Stained})^2 + (b_{Clean} - b_{Stained})^2]}$$

And

$$\Delta E_{post-wash} = \sqrt{[(L_{Clean} - L_{Washed})^2 + (a_{Clean} - a_{Washed})^2 + (b_{Clean} - b_{Washed})^2]}$$

| | Enzyme in the detergent composition | SRI | SE (n=4) | p-value vs A (95%) | p-value vs B (95%) |
|---|---|---|---|---|---|
| A | Nil | 69.7 | 0.9 | | |
| B | CZ08612 endo-β-1,3-glucanase | 71.8 | 1.0 | 0.158 | |
| C | *Alkalitalea saponilacus* endo-β-1,3-glucanase (SEQ ID NO: 1) | 87.1 AB | 1.9 | 0.001 | 0.001 |

SE is the standard error. Statistical significance is determined using Student's t-test: a p value <0.05 implies a significant difference at 95% confidence level. Where a letter is shown after the SRI result, that test leg was found to have significantly improved cleaning versus the legs denoted by the letters.

As it can be seen from the data above, there is a is statistically significant (P-value = 0.001) difference in performance of SEQ ID NO: 1 and CZ08612 endo-β-1,3-glucanase. This shows that detergent compositions comprising the endo-β-1,3-glucanase according to the invention (*Alkalitalea saponilacus* endo-β-1,3-glucanase (SEQ ID NO: 1)) provides better removal of foundation make-up from cotton fabrics than an endo-β-1,3-glucanase outside the scope of the invention (CZ08612, NZYtech).

Example 3

The removal of chocolate pudding stains from cotton fabrics was evaluated using a liquid laundry detergent comprising a mannanase according to the invention (*Alkalitalea saponilacus* GH26 mannanase (SEQ ID NO: 49) and a liquid detergent comprising a mannanase outside the scope of the invention (Mannaway GH5).

Chocolate pudding stains (CFT - Vlaardingen, The Netherlands, KC-S-69, batch:022) were cut into 5cm x 5cm swatches and the color of stains and white background fabric were image analysed for CIELab values (DigiEye, VeriVide, Leicester, U.K.) before testing.

Stain removal testing was conducted using a Tergotometer to simulate the washing of fabrics in a washing machine. Pots were filled with 1L of water (20 grains per US gallon) heated to 35°C, and 2.7mL/L Fairy UK non-bio liquid detergent (Purchased from Asda Stores, UK, batch 1235030375) was added to each pot. Two stain swatches were added to each pot, together with 10g of SBL2004 soil sheets cut into 5cm x 5cm swatches (WFK-Testgewebe GmbH, product code 10996) and sufficient 5cm x 5cm swatches of clean white knitted cotton ballast (Warwick Equest Ltd) to reach a total fabric load of 60g. The addition of 1mg active GH5 mannanase (Mannaway 25L, batch KHP30089, Novozymes A/S, Bagsvaerd, Denmark) and 1mg active GH26 mannanase (*Alkalitalea saponilacus,* Northumbria University, Newcastle-Upon-Tyne, UK, (SEQ ID 49)) were added via a pipette to each respective wash pot, resulting in a wash concentration of 0.5ppm active enzyme. The agitation was then initiated at 300rpm for 40 minutes and the temperature was maintained at 35°C for the duration of the test. After 40 minutes, the wash water was drained, and the fabrics rinsed twice for five minutes in 1L 15°C water (20 grains per US gallon) at 208rpm, before placing the washed stain swatches flat on a rack to dry under ambient conditions.

This process was repeated three more times, resulting in a total of eight washed stain swatches per treatment, i.e. four external replicates, each comprising two swatches of one stains. The washed stains were analysed for CIELab as before, and Stain Removal Index (SRI) calculated using the following equation:

$$SRI = \left(\frac{\Delta E_{pre-wash} - \Delta E_{post-wash}}{\Delta E_{pre-wash}}\right) x\ 100$$

Where

$$\Delta E_{pre-wash} = \sqrt{[(L_{Clean} - L_{Stained})^2 + (a_{Clean} - a_{Stained})^2 + (b_{Clean} - b_{Stained})^2]}$$

And

$$\Delta E_{post-wash} = \sqrt{[(L_{Clean} - L_{Washed})^2 + (a_{Clean} - a_{Washed})^2 + (b_{Clean} - b_{Washed})^2]}$$

| | Enzyme in the detergent composition | SRI | SE (n=4) | p-value vs A (90%) | p-value vs B (90%) |
|---|---|---|---|---|---|
| A | Nil | 49.6 | 1.8 | | |
| B | Mannaway GH5 | 50.7 | 3.1 | 0.773 | |
| C | *Alkalitalea saponilacus* GH26 mannanase (SEQ ID NO: 49) | 60.1 AB | 1.9 | 0.007 | 0.052 |

SE is the standard error. Statistical significance is determined using Student's t-test: a p value <0.1 implies a significant difference at 90% confidence level. Where a letter is shown after the SRI result, that test leg was found to have significantly improved cleaning versus the legs denoted by the letters.

As it can be seen from the table above, the detergent composition comprising the mannanase according to the invention (*Alkalitalea saponilacus* GH26 mannanase (SEQ ID NO: 49)) provides better removal of chocolate pudding stains on cotton fabrics than a mannanase outside the scope of the invention (Mannaway GH5).

Example 4

The removal of oatmeal soil from stainless steel with a combination of protease (IFF) and amylase (IFF) and an endo-β-1,3-glucanase according to the invention (*Alkalitalea saponilacus* endo-β-1,3-glucanase (SEQ ID NO: 1)) was compared with the removal with a combination of protease (IFF) and amylase (IFF) only.

Oatmeal soil consisting of instant oats (Quaker) and UHT full fat milk (Asda) was prepared by heating the mixture in a microwave for 2 min and blending the mixture in a liquidizer, supplied by Russell Hobbs limited UK, for 1 min. Soil mixtures were applied onto the centre of 3x3 cm stainless steel tokens and left at ambient room temperature overnight.

Soil removal testing was conducted using soiled tokens in 6-well plates on an incubating rocking shaker supplied by OHAUS corporation, USA.

Soiled tokens were incubated in wells containing 5 ml buffer consisting of 1.52g/L Sodium hydrogen carbonate (VWR) and 8.68g/L Sodium carbonate (Newport Industries). 5ppm of protease (IFF) and 5ppm of amylase (IFF) were tested *vs.* 5ppm of protease (IFF), 2.5ppm of amylase (IFF) and 2.5ppm endo-β-1,3-glucanase (*Alkalitalea saponilacus,* SEQ ID NO: 1). Reactions were carried out for 45 min at 40°C, in buffered solution pH 10.5. Washed tokens were removed from their respective wells and dried at 80°C for 30min.

Once dry, soil removal from token surfaces was calculated by gravimetric analysis using weights of soiled tokens before and after washing. Percentage soil removal was calculated by dividing grams of soil removed, by grams of soil remaining, multiplied by 100. 2 external replicates were carried out for each of the treatments and differences to nil enzyme were plotted, with standard error indicated

Results

| | Δ Soil removal to nil (%) | SE (n=2) | p-value vs. Protease 5ppm + Amylase 5ppm |
|---|---|---|---|
| Protease (IFF) 5ppm + Amylase (IFF) 5ppm | 16.92 | 3.51 | - |
| Protease (IFF) 5ppm + Amylase (IFF) 2.5ppm + *Alkalitalea saponilacus* endo-β-1,3-glucanase 2.5ppm | 25.57 | 3.51 | 0.0299 |

SE is the standard error. Statistical significance is determined using Student's t-test: a p value <0.05 implies a significant difference at 95% confidence level. Mean ± standard error is shown for each leg.

As it can be seen from the data above, a combination of protease (IFF) and amylase (IFF) and the endo-β-1,3-glucanase according to the invention (*Alkalitalea saponilacus* endo-β-1,3-glucanase (SEQ ID NO: 1)) provides better oatmeal soil removal from a stainless steel surface than a combination of protease (IFF) and amylase (IFF) alone.

DETERGENT EXAMPLES

Examples 5-10. Granular laundry detergent compositions designed for hand washing or top-loading washing machines.

| | 1 (wt %) | 2 (wt %) | 3 (wt %) | 4 (wt %) | 5 (wt %) | 6 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |

(continued)

| | 1 (wt%) | 2 (wt %) | 3 (wt%) | 4 (wt%) | 5 (wt%) | 6 (wt%) |
|---|---|---|---|---|---|---|
| 1.6R Silicate (SiO2:Na2O at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer[1] | 0.1 | 0.2 | 0.0 | 0.0 | 0.0 | 0.0 |
| Carboxymethyl cellulose | 1 | 0.3 | 1 | 1 | 1 | 1 |
| Protease (Savinase®, 32.89 mg active/g) | 0.1 | 0.1 | 0.1 | 0.1 | | 0.1 |
| [5]DNase as defined herein (mg active per 100g composition) | 2.0 | 0 | 4.4 | 0 | 0.4 | 0 |
| Lipase - Lipex® (18 mg active /g) | 0.03 | 0.07 | 0.3 | 0.1 | 0.07 | 0.4 |
| [4]Amylase Stainzyme® Plus (mg active) | 3.0 | 5.0 | 3.0 | 2.2 | 6.0 | 6.0 |
| [6]Alginate lyase as defined herein (mg active per 100g of detergent) | 12.0 | 15.0 | 3.2 | 4.3 | 9.2 | 17.0 |
| [7]Hexosaminidase as defined herein (mg active per 100g composition) | 2.0 | 6.0 | 5.6 | 2.2 | 4.0 | 1.5 |
| [8]Pel-ase as defined herein (mg active per 100g composition) | 4.3 | 0.0 | 3.4 | 7.2 | 1.9 | 3.3 |
| [9]Psl-ase as defined herein (mg active per 100g composition) | 3.2 | 3.2 | 0.0 | 1.2 | 0.0 | 5.3 |
| [10]Endo-beta-1,3(4)-glucanase as defined herein (mg active per 100g composition) | 9.1 | 5.4 | 0.0 | 4.3 | 3.0 | 3.1 |
| "Enzyme or variant of SEQ ID NO: 1-190 in accordance with the invention (mg active per 100g composition) | 3.0 | 4.7 | 7.1 | 21.6 | 15.9 | 5.0 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| MgSO4 | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

Examples 7-13. Granular laundry detergent compositions designed for front-loading automatic washing machines.

| | 7 (wt%) | 8 (wt%) | 9 (wt%) | 10 (wt%) | 11 (wt%) | 12 (wt%) | 13 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |
| AE7 | 2.2 | 0 | 3.2 | 0 | 0 | 0 | 1 |
| C10-12 Dimethyl hydroxyethylammonium chloride | 0.75 | 0.94 | 0.98 | 0.98 | 0 | 0 | 0 |
| Crystalline layered silicate (δ-Na2Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2:1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Protease - Purafect® (84 mg active/g) | 0.2 | 0.2 | 0.3 | 0.15 | 0.12 | 0.13 | 0.18 |
| Lipase - Lipex® (18.00 mg active/g) | 0.05 | 0.15 | 0.1 | 0 | 0 | 0 | 0.1 |
| Cellulase - CellucleanTM (15.6 mg active/g) | 0 | 0 | 0 | 0 | 0.1 | 0.1 | 0 |
| [4]Amylase Stainzyme® Plus (mg active) | 4.0 | 5.0 | 10 | 2.2 | 4.4 | 1.5 | 1.5 |
| Mannanase - Mannaway® (4mg active/g) | 0.05 | 0.1 | 0 | 0.05 | 0.1 | 0 | 0.1 |
| [5]DNase as defined herein (mg active per 100g detergent) | 1.0 | 0 | 2.0 | 0 | 4.0 | 0 | 0 |
| [6]Alginate lyase as defined herein (mg active per 100g of detergent) | 3.3 | 9.2 | 12.0 | 4.7 | 3.7 | 13.2 | 3.3 |
| [7]Hexosaminidase as defined herein (mg active per 100g detergent) | 3.0 | 5.0 | 8.0 | 2.2 | 4.0 | 1.5 | 0.1 |
| [8]Pel-ase as defined herein (mg active per 100g composition) | 3.2 | 5.3 | 0.0 | 0.0 | 0.0 | 3.0 | 3.3 |
| [9]Psl-ase as defined herein (mg active per 100g composition) | 0.0 | 0.0 | 0.0 | 3.2 | 8.2 | 1.1 | 0.9 |
| [10]Endo-beta-1,3(4)-glucanase as defined herein (mg active per 100g composition) | 0.0 | 0.0 | 3.2 | 1.0 | 3.3 | 2.2 | 3.2 |
| [11] Enzyme or variant of SEQ ID NO: 1-190 in accordance with the invention (mg active per 100g composition) | 3.0 | 4.7 | 7.1 | 8.9 | 6.4 | 5.0 | 10.0 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane di phosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |

(continued)

| | 7 (wt%) | 8 (wt%) | 9 (wt%) | 10 (wt%) | 11 (wt%) | 12 (wt%) | 13 (wt%) |
|---|---|---|---|---|---|---|---|
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.0007 | 0.0012 | 0.0007 | 0 | 0 | 0 | 0 |
| S-ACMC | 0.01 | 0.01 | 0 | 0.01 | 0 | 0 | 0 |
| Direct Violet 9 (active) | 0 | 0 | 0.0001 | 0.0001 | 0 | 0 | 0.001 |
| Sulfate/ Water & Miscellaneous | Balance | | | | | | |
| *DNase is shown as mgs of active enzyme per 100g of detergent. | | | | | | | |

Examples 14-21. Heavy Duty Liquid laundry detergent compositions

| | 14 (wt%) | 15 (wt%) | 16 (wt%) | 17 (wt%) | 18 (wt%) | 19 (wt%) | 20 (wt%) | 21 (wt%) |
|---|---|---|---|---|---|---|---|---|
| C12-15 Alkylethoxy(1.8) sulfate | 14.7 | 11.6 | 0.0 | 16.3 | 0.0 | 17.3 | 20 | 12 |
| C11.8 Alkylbenzene sulfonate | 4.3 | 11.6 | 8.3 | 7.8 | 11.7 | 7.8 | 7 | 0 |
| C16-17 Branched alkyl sulfate | 1.7 | 1.29 | 0.0 | 3.09 | 0.0 | 3.3 | 0 | 0 |
| C12-14 Alkyl -9-ethoxylate | 0.9 | 1.07 | 0.0 | 1.31 | 0.0 | 1.31 | 5 | 0 |
| C12 dimethylamine oxide | 0.6 | 0.64 | 0.0 | 1.03 | 0.0 | 1.03 | 2 | 3 |
| Citric acid | 3.5 | 0.65 | 3 | 0.66 | 2.27 | 0.67 | 1 | 0 |
| C12-18 fatty acid | 1.5 | 2.32 | 3.6 | 1.52 | 0.82 | 1.52 | 1 | 0 |
| Sodium Borate (Borax) | 2.5 | 2.46 | 1.2 | 2.53 | 0.0 | 2.53 | 0 | 1 |
| Sodium C12-14 alkyl ethoxy 3 sulfate | 0.0 | 0.0 | 2.9 | 0.0 | 3.9 | 0.0 | 0 | 14 |
| C14-15 alkyl 7-ethoxylate | 0.0 | 0.0 | 4.2 | 0.0 | 1.9 | 0.0 | 0 | 4.2 |
| C12-14 Alkyl -7-ethoxylate | 0.0 | 0.0 | 1.7 | 0.0 | 0.5 | 0.0 | 0 | 1.7 |
| Ca chloride dihydrate | 0.0 | 0.0 | 0.0 | 0.0 | 0.045 | 0.0 | 0 | 0 |
| Ca formate | 0.09 | 0.09 | 0.0 | 0.09 | 0.0 | 0.09 | 0.09 | 0 |
| A compound: bis((C2H5O)(C2H4O)n)(CH 3)-N+-CxH2x-N+-(CH3)-bis((C2H5O)(C2H4O)n); n is 20 to 30; x is 3 to 8, optionally sulphated or sulphonated | 0.0 | 0.0 | 1.2 | 0.0 | 0.66 | 0.0 | 0.0 | 1.2 |
| Random graft co-polymer[1] | 0.0 | 1.46 | 0.5 | 0.0 | 0.83 | 0.0 | 0.0 | 0.5 |
| Ethoxylated Polyethylenimine[2] | 1.5 | 1.29 | 0.0 | 1.44 | 0.0 | 1.44 | 1.44 | 0.0 |
| Diethylene triamine pentaacetic acid | 0.34 | 0.64 | 0.0 | 0.34 | 0.0 | 0.34 | 0.34 | 0.0 |
| Diethylene triamine penta (methylene phosphonic acid) | 0.0 | 0.0 | 0.3 | 0.0 | 0.3 | 0.0 | 0.0 | 0.3 |
| 1-hydroxyethyidene-1,1-diphosphonic acid | 0.0 | 0.0 | 0.0 | 0.0 | 0.18 | 0.0 | 0.0 | 0.0 |
| Dihydroxybenzene -3,5-disulfonic acid disodium salt hydrate | 0.0 | 0.0 | 0.0 | 0.0 | 0.0 | 0.19 | 0.19 | 0.0 |
| Tinopal AMS-GX | 0.0 | 0.06 | 0.0 | 0.0 | 0.0 | 0.29 | 0.29 | 0.0 |

(continued)

| | | | | | | | | |
|---|---|---|---|---|---|---|---|---|
| Tinopal CBS-X | 0.2 | 0.17 | 0.0 | 0.29 | 0.0 | 0.0 | 0.0 | 0.0 |
| Tinopal TAS-X B36 | 0.0 | 0.0 | 0.0 | 0.0 | 0.091 | 0.0 | 0.0 | 0.0 |
| Amphiphilic alkoxylated grease cleaning polymer[3] | 1.28 | 1 | 0.4 | 1.93 | 0.0 | 1.93 | 1.93 | 0.4 |
| CHEC | 0.0 | 0.0 | 0.2 | 0.0 | 0.0 | 0.0 | 0.0 | 0.2 |
| Ethanol | 2 | 1.58 | 1.6 | 5.4 | 1.2 | 3.57 | 0 | 1.6 |
| Propylene Glycol | 3.9 | 3.59 | 1.3 | 4.3 | 0.0 | 3.8 | 3.8 | 1.3 |
| Diethylene glycol | 1.05 | 1.54 | 0.0 | 1.15 | 0.0 | 1.15 | 1.15 | 0.0 |
| Polyethylene glycol | 0.06 | 0.04 | 0.0 | 0.1 | 0.0 | 0.1 | 0.1 | 0.0 |
| [4]Amylase Amplify® (mg active) | 8.0 | 7.0 | 2.5 | 4.0 | 3.0 | 1.7 | 3 | 2.5 |
| [5]DNase (mg active per 100g detergent) | 0 | 2.0 | 0 | 2.0 | 1.25 | 1.0 | 5 | 0 |
| [6]Alginate lyase as defined herein (mg active per 100g of detergent) | 3.2 | 4.1 | 7.9 | 12.4 | 3.7 | 5.0 | 17.3 | 2.1 |
| [7]Hexosaminidase as defined herein (mg active per 100g detergent) | 7.0 | 3.0 | 8.0 | 2.2 | 1.25 | 10 | 0.1 | 2.5 |
| [8]Pel-ase as defined herein (mg active per 100g composition) | 2.2 | 0.0 | 1.2 | 0.8 | 0.10 | 8.3 | 2.2 | 3.9 |
| [9]Psl-ase as defined herein (mg active per 100g composition) | 3.4 | 4.4 | 0.0 | 0.0 | 0.0 | 4.3 | 5.3 | 3.2 |
| [10]Endo-beta-1,3(4)-glucanase as defined herein (mg active per 100g composition) | 2.2 | 0.0 | 1.4 | 5.3 | 2.2 | 3.3 | 4.5 | 3.3 |
| [11] Enzyme or variant of SEQ ID NO: 1-190 in accordance with the invention (mg active per 100g composition) | 3.4 | 8.2 | 16.2 | 12.8 | 9.5 | 4.1 | 3.9 | 18.2 |
| Monoethanolamine | 3.05 | 2.41 | 0.4 | 1.26 | 0.31 | 1.13 | 1.13 | 0.4 |
| NaOH | 2.44 | 1.8 | 0.0 | 3.01 | 3.84 | 0.24 | 0.24 | 0.0 |
| Sodium Cumene Sulphonate | 0.0 | 0.0 | 1 | 0.0 | 0.95 | 0.0 | 0.0 | 1 |
| Sodium Formate | 0.0 | 0.11 | 0.0 | 0.09 | 0.2 | 0.12 | 0.12 | 0.0 |
| Polyethoxylated azo thiophene dye | 0.001 | 0.001 | 0.001 | 0.05 | 0.0001 | 0.0001 | 0.0001 | 0.001 |
| Water, Aesthetics (Dyes, perfumes) and Minors (Enzymes including lipase, protease, additional amylase each at 0.2% active protein, solvents, structurants) | balance | | | | | | | |

Examples 22-28. Unit Dose Laundry detergent compositions. Such unit dose formulations can comprise one or multiple compartments.

| | 22 (wt%) | 23 (wt%) | 24 (wt%) | 25 (wt%) | 26 (wt%) | 27 (wt%) | 28 (wt%) |
|---|---|---|---|---|---|---|---|
| Alkylbenzene sulfonic acid | 14.5 | 14.5 | 14.5 | 14.5 | 14.5 | 23 | 23 |

(continued)

| | 22 (wt%) | 23 (wt%) | 24 (wt%) | 25 (wt%) | 26 (wt%) | 27 (wt%) | 28 (wt%) |
|---|---|---|---|---|---|---|---|
| C12-18 alkyl ethoxy 2.5 sulfate | 7.5 | 7.5 | 7.5 | 7.5 | 7.5 | 16 | 16 |
| C12-18 alkyl 7-ethoxylate | 13.0 | 13.0 | 13.0 | 13.0 | 13.0 | 3.1 | 3.8 |
| C14-15 alkyl 9-ethoxylate | 0 | 0 | 0 | 0 | 0 | 1 | 0 |
| Citric Acid | 0.6 | 0.6 | 0.6 | 0.6 | 0.6 | 0.9 | 0.7 |
| Fatty Acid | 14.8 | 14.8 | 14.8 | 14.8 | 14.8 | 6.5 | 6 |
| Amylase (mg active) | 6 | 12 | 8 | 2 | 10 | 2 | 2 |
| Ethoxylated Polyethylenimine[2] | 4.0 | 4.0 | 4.0 | 4.0 | 4.0 | 4.0 | 4.0 |
| Protease (Purafect Prime®, 40.6 mg active/g) | 1.4 | 2.0 | 0.9 | 1.2 | 0 | 1 | 1 |
| Cellulase (Celluclean, active protein) | 0.1 | 0.2 | 0.0 | 0.0 | 0.1 | 0 | 0 |
| [5]DNase described herein (mg active per 100g detergent) | 3.0 | 2.0 | 1.0 | 2.0 | 2.0 | 1 | 1 |
| [4]Amylase Amplify® (active protein) | 0.0 | 0.0 | 0.1 | 0.2 | 0.1 | 0.5 | 0.5 |
| [6]Alginate lyase as defined herein (mg active per 100g of detergent) | 2.2 | 3.1 | 2.3 | 5.2 | 5.3 | 12.2 | 5.4 |
| [7]Hexosaminidase as defined herein (mg active per 100g detergent) | 5.0 | 10 | 0.0 | 2.2 | 1.2 | 3 | 0.9 |
| [8]Pel-ase as defined herein (mg active per 100g composition) | 1.3 | 0.0 | 2.45 | 8.3 | 4.0 | 3.2 | 5.3 |
| [9]Psl-ase as defined herein (mg active per 100g composition) | 0.0 | 3.4 | 7.3 | 3.2 | 1.5 | 4.7 | 0.0 |
| [10]Endo-beta-1,3(4)-glucanase as defined herein (mg active per 100g composition) | 3.4 | 3.3 | 3.4 | 0.0 | 3.1 | 1.9 | 6.3 |
| [11] Enzyme or variant of SEQ ID NO: 1-190 in accordance with the invention (mg active per 100g composition) | 3.0 | 4.1 | 12.2 | 6.8 | 15.9 | 2.2 | 8.4 |
| Hydroxyethane diphosphonic acid | 1.2 | 1.2 | 1.2 | 1.2 | 1.2 | 0 | 2.3 |
| Brightener | 0.3 | 0.3 | 0.3 | 0.3 | 0.3 | 0.2 | 0.2 |
| P-diol | 15.8 | 13.8 | 13.8 | 13.8 | 13.8 | 12.2 | 12.2 |
| Glycerol | 6.1 | 6.1 | 6.1 | 6.1 | 6.1 | 4.0 | 3.8 |
| MEA | 8.0 | 8.0 | 8.0 | 8.0 | 8.0 | 8.6 | 10.2 |
| TIPA | 0.0 | 0.0 | 2.0 | 0.0 | 0.0 | 0.0 | 0.0 |
| TEA | 0.0 | 2.0 | 0.0 | 0.0 | 0.0 | 0.0 | 0.0 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| Water | 10 | 10 | 10 | 10 | 10 | 10 | 10 |
| Structurant | 0.14 | 0.14 | 0.14 | 0.14 | 0.14 | 0.14 | 0.14 |
| Perfume | 1.9 | 1.9 | 1.9 | 1.9 | 1.9 | 1.9 | 1.9 |
| Buffers (monoethanolamine) | To pH 8.0 | | | | | | |

(continued)

| | |
|---|---|
| Solvents (1,2 propanediol, ethanol) & minors | To 100% |

Example 29. Multiple Compartment Unit Dose Composition

Multiple compartment unit dose laundry detergent formulations of the present invention are provided below. In these examples the unit dose has three compartments, but similar compositions can be made with two, four or five compartments. The film used to encapsulate the compartments is polyvinyl alcohol.

| Base composition 1 | 29 (wt%) |
|---|---|
| Glycerol (min 99) | 5.3 |
| 1,2-propanediol | 10.0 |
| Citric Acid | 0.5 |
| Monoethanolamine | 10.0 |
| Caustic soda | - |
| Dequest 2010 | 1.1 |
| Potassium sulfite | 0.2 |
| [5]DNase as defined herein (mg active) | 4.0 |
| [6]Alginate lyase as defined herein (mg active per 100g of detergent) | 12.2 |
| [7]Hexosaminidase as defined herein (mg active per 100g detergent) | 7.0 |
| [8]Pel-ase as defined herein (mg active per 100g composition) | 2.3 |
| [9]Psl-ase as defined herein (mg active per 100g composition) | 4.2 |
| [10]Endo-beta-1,3(4)-glucanase as defined herein (mg active per 100g composition) | 5.4 |
| [11]Enzyme or variant of SEQ ID NO: 1-190 in accordance with the invention (mg active per 100g composition) | 10.2 |
| Nonionic Marlipal C24EO7 | 20.1 |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

Multi-compartment formulations

| Composition | 1 | | | 2 | | |
|---|---|---|---|---|---|---|
| Compartment | A | B | C | A | B | C |
| Volume of each compartment | 40 ml | 5 ml | 5 ml | 40 ml | 5 ml | 5 ml |

(continued)

| Active material in Wt.% | | | | | | |
|---|---|---|---|---|---|---|
| Perfume | 1.6 | 1.6 | 1.6 | 1.6 | 1.6 | 1.6 |
| Dyes | < 0.01 | < 0.01 | < 0.01 | < 0.01 | < 0.01 | < 0.01 |
| TiO2 | 0.1 | - | - | - | 0.1 | - |
| Sodium Sulfite | 0.4 | 0.4 | 0.4 | 0.3 | 0.3 | 0.3 |
| Acusol 305 | 1.2 | | | 2 | - | - |
| Hydrogenated castor oil | 0.14 | 0.14 | 0.14 | 0.14 | 0.14 | 0.14 |
| Base Composition 1 | Add to 100% | Add to 100% | Add to 100% | Add to 100% | Add to 100% | Add to 100% |

Raw Materials and Notes for Composition Examples 5-29

Linear alkylbenzenesulfonate having an average aliphatic carbon chain length C11-C18
C12-18 Dimethylhydroxyethyl ammonium chloride
AE3S is C12-15 alkyl ethoxy (3) sulfate
AE7 is C12-15 alcohol ethoxylate, with an average degree of ethoxylation of 7
AE9 is C12-16 alcohol ethoxylate, with an average degree of ethoxylation of 9
HSAS is a mid-branched primary alkyl sulfate with carbon chain length of about 16-17 as disclosed in US 6,020,303 and US 6,060,443
Polyacrylate MW 4500 is supplied by BASF
Carboxymethyl cellulose is Finnfix® V supplied by CP Kelco, Amhem, Netherlands
CHEC is a cationically modified hydroxyethyl cellulose polymer.
Phosphonate chelants are, for example, diethylenetetraamine pentaacetic acid (DTPA) Hydroxyethane di phosphonate (HEDP)
Savinase®, Natalase®, Stainzyme®, Lipex®, CellucleanTM, Mannaway® and Whitezyme® are all products of Novozymes, Bagsvaerd, Denmark.
Purafect®, Purafect Prime® are products of Genencor International, Palo Alto, California, USA
Fluorescent Brightener 1 is Tinopal® AMS, Fluorescent Brightener 2 is Tinopal® CBS-X, Direct Violet 9 is Pergasol® Violet BN-Z NOBS is sodium nonanoyloxybenzenesulfonate
TAED is tetraacetylethylenediamine
S-ACMC is carboxymethylcellulose conjugated with C.I. Reactive Blue 19product name AZO-CM-CELLULOSE
Soil release agent is Repel-o-tex® PF
Acrylic Acid/Maleic Acid Copolymer is molecular weight 70,000 and acrylate:maleate ratio 70:30
EDDS is a sodium salt of ethylenediamine-N,N'-disuccinic acid, (S,S) isomer Suds suppressor agglomerate is supplied by Dow Coming, Midland, Michigan, USA
HSAS is mid-branched alkyl sulfate
Liquitint® Violet CT polymeric hueing dye, supplied by Milliken, Spartanburg, South Carolina, USA Polyethoxylated azo thiophene dye is Violet DD™ polymeric hueing dye, supplied by Milliken, Spartanburg, South Carolina, USA.

[1] Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
[2] Polyethyleneimine (MW = 600) with 20 ethoxylate groups per -NH.
[3] Amphiphilic alkoxylated polymer is a polyethylenimine (MW 600), prepared from a polymer that is derivatised to contain 24 ethoxylate groups per -NH and 16 Propoxylate groups per -NH.
[4]Amylase is shown as mgs of active enzyme per 100g of detergent.
[5]DNase as described herein (in all of these examples is shown as mgs of active enzyme per 100g of detergent). DNase may comprise minor amounts of super oxide dismutase impurity.
[6]Alginate lyase as described herein (in all examples shown as mgs of active enzyme per 100g of detergent)
[7]Hexosaminidase as described herein (in all examples shown as mgs of active enzyme per 100g of detergent)

[8]Pel-ase as described herein (in all examples shown as mgs of active enzyme per 100g of detergent)
[9]Psl-ase as described herein (in all examples shown as mgs of active enzyme per 100g of detergent)
[10]Endo-beta-1,3(4)-glucanase as described herein (in all examples shown as mgs of active enzyme per 100g of detergent).[a] Proxel GXL, 20% aqueous dipropylene glycol solution of 1,2-benzisothiazolin-3-one, supplied by Lonza.
[11]Enzyme endogenous to *Alkalitalea saponilacus* or variant thereof in accordance with SEQ ID NO: 1-190, as defined by the claims herein (in all examples shown as mg of active enzyme per 100g of composition)

[b] N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.
[c] MP10®, supplied by Dow Corning, 8% activity
[d] as described in US 8,765,659, expressed as 100% encapsulated perfume oil
[e] Rheovis® CDE, cationic polymeric thickener supplied by BASF
[f] N,N-dimethyl octanamide and N,N-dimethyl decanamide in about a 55:45 weight ratio, tradename Steposol® M-8-10 from the Stepan Company

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An alkaline cleaning composition comprising:

   (a) from 0.00005 to 5% by weight thereof of an enzyme endogenous to *Alkalitalea saponilacus* or a variant thereof selected from the group consisting of:

      (i) a glycosyl hydrolase having at least 35% sequence identity to SEQ ID NO: 1, at least 45% sequence identity to SEQ ID NO: 2, at least 55% sequence identity to SEQ ID NO: 3, at least 50% sequence identity to SEQ ID NO: 4, at least 60% sequence identity to SEQ ID NO: 5, at least 50% sequence identity to SEQ ID NO: 6, at least 50% sequence identity to SEQ ID NO: 7, at least 50% sequence identity to SEQ ID NO: 8, at least 40% sequence identity to SEQ ID NO: 9, at least 45% sequence identity to SEQ ID NO: 10, at least 60% sequence identity to SEQ ID NO: 11, at least 60% sequence identity to SEQ ID NO: 12, at least 60% sequence identity to SEQ ID NO: 13, at least 55% sequence identity to SEQ ID NO: 14, at least 50% sequence identity to SEQ ID NO: 15, at least 35% sequence identity to SEQ ID NO: 16, at least 55% sequence identity to SEQ ID NO: 17, at least 50% sequence identity to SEQ ID NO: 18, at least 40% sequence identity to SEQ ID NO: 19, at least 30% sequence identity to SEQ ID NO: 20, at least 55% sequence identity to SEQ ID NO: 21, at least 40% sequence identity to SEQ ID NO: 22, at least 40% sequence identity to SEQ ID NO: 23, at least 40% sequence identity to SEQ ID NO: 24, at least 30% sequence identity to SEQ ID NO: 25, at least 50% sequence identity to SEQ ID NO: 26, at least 50% sequence identity to SEQ ID NO: 27, at least 35% sequence identity to SEQ ID NO: 28, at least 45% sequence identity to SEQ ID NO: 29, at least 45% sequence identity to SEQ ID NO: 30, at least 40% sequence identity to SEQ ID NO: 31, at least 60% sequence identity to SEQ ID NO: 32, at least 45% sequence identity to SEQ ID NO: 33, at least 35% sequence identity to SEQ ID NO: 34, at least 50% sequence identity to SEQ ID NO: 35, at least 60% sequence identity to SEQ ID NO: 36, at least 40% sequence identity to SEQ ID NO: 37, at least 40% sequence identity to SEQ ID NO: 38, at least 50% sequence identity to SEQ ID NO: 39, at least 55% sequence identity to SEQ ID NO: 40, at least 40% sequence identity to SEQ ID NO: 41, at least 40% sequence identity to SEQ ID NO: 42, at least 40% sequence identity to SEQ ID NO: 43, at least 55% sequence identity to SEQ ID NO: 44, at least 35% sequence identity to SEQ ID NO: 45, at least 50% sequence identity to SEQ ID NO: 46, at least 40% sequence identity to SEQ ID NO: 47, at least 35% sequence identity to SEQ ID NO: 48, at least 55% sequence identity to SEQ ID NO: 49, at least 50% sequence identity to SEQ ID NO: 50, at least 55% sequence identity to SEQ ID NO: 51, at least 60% sequence identity to SEQ ID NO: 52, at least 30% sequence identity to SEQ ID NO: 53, at least 65% sequence identity to SEQ ID NO: 54, at least 40% sequence identity to SEQ ID NO: 55, at least 65% sequence identity to SEQ ID NO: 56, at least 35% sequence identity to SEQ ID NO: 57, at least 60% sequence identity to SEQ ID NO: 58, at least 70% sequence identity to SEQ ID NO: 59, at least 60% sequence identity to SEQ ID NO: 60, at least 40% sequence identity to SEQ ID

NO: 61, at least 30% sequence identity to SEQ ID NO: 62, at least 35% sequence identity to SEQ ID NO: 63, at least 35% sequence identity to SEQ ID NO: 64, at least 45% sequence identity to SEQ ID NO: 65, at least 60% sequence identity to SEQ ID NO: 66, at least 60% sequence identity to SEQ ID NO: 67, at least 45% sequence identity to SEQ ID NO: 68, at least 35% sequence identity to SEQ ID NO: 69, at least 55% sequence identity to SEQ ID NO: 70, at least 45% sequence identity to SEQ ID NO: 71, at least 40% sequence identity to SEQ ID NO: 72, at least 65% sequence identity to SEQ ID NO: 73, at least 50% sequence identity to SEQ ID NO: 74, at least 85% sequence identity to SEQ ID NO: 75, at least 85% sequence identity to SEQ ID NO: 76, at least 35% sequence identity to SEQ ID NO: 77, at least 50% sequence identity to SEQ ID NO: 78, at least 50% sequence identity to SEQ ID NO: 79, at least 45% sequence identity to SEQ ID NO: 80, at least 55% sequence identity to SEQ ID NO: 81, at least 40% sequence identity to SEQ ID NO: 82, at least 35% sequence identity to SEQ ID NO: 83, at least 45% sequence identity to SEQ ID NO: 84, at least 50% sequence identity to SEQ ID NO: 85, at least 60% sequence identity to SEQ ID NO: 86, at least 50% sequence identity to SEQ ID NO: 87, at least 50% sequence identity to SEQ ID NO: 88, at least 55% sequence identity to SEQ ID NO: 89, at least 50% sequence identity to SEQ ID NO: 90, at least 75% sequence identity to SEQ ID NO: 91, at least 55% sequence identity to SEQ ID NO: 92, at least 45% sequence identity to SEQ ID NO: 93, at least 50% sequence identity to SEQ ID NO: 94, at least 55% sequence identity to SEQ ID NO: 95, at least 55% sequence identity to SEQ ID NO: 96, at least 45% sequence identity to SEQ ID NO: 97, at least 45% sequence identity to SEQ ID NO: 98, at least 55% sequence identity to SEQ ID NO: 99, at least 55% sequence identity to SEQ ID NO: 100, at least 30% sequence identity to SEQ ID NO: 101, at least 35% sequence identity to SEQ ID NO: 102, at least 45% sequence identity to SEQ ID NO: 103, at least 55% sequence identity to SEQ ID NO: 104, at least 50% sequence identity to SEQ ID NO: 105,

(ii) a polysaccharide lyase or a variant thereof having at least 55% sequence identity to SEQ ID NO: 106, at least 50% sequence identity to SEQ ID NO: 107, at least 45% sequence identity to SEQ ID NO: 108, at least 55% sequence identity to SEQ ID NO: 109, at least 40% sequence identity to SEQ ID NO: 110, at least 50% sequence identity to SEQ ID NO: 111,

(iii) an esterase / lipase or a variant thereof having at least 50% sequence identity to SEQ ID NO: 112, at least 65% sequence identity to SEQ ID NO: 113, at least 45% sequence identity to SEQ ID NO: 114, at least 45% sequence identity to SEQ ID NO: 115, at least 40% sequence identity to SEQ ID NO: 116, at least 40% sequence identity to SEQ ID NO: 117, at least 70% sequence identity to SEQ ID NO: 118, at least 30% sequence identity to SEQ ID NO: 119, at least 30% sequence identity to SEQ ID NO: 120, at least 30% sequence identity to SEQ ID NO: 121, at least 50% sequence identity to SEQ ID NO: 122, at least 55% sequence identity to SEQ ID NO: 123, at least 35% sequence identity to SEQ ID NO: 124,

(iv) a nuclease or a variant thereof having at least 50% sequence identity to SEQ ID NO: 125, at least 50% sequence identity to SEQ ID NO: 126, at least 35% sequence identity to SEQ ID NO: 127, at least 40% sequence identity to SEQ ID NO: 128, at least 40% sequence identity to SEQ ID NO: 129, at least 60% sequence identity to SEQ ID NO: 130, at least 55% sequence identity to SEQ ID NO: 131, at least 30% sequence identity to SEQ ID NO: 132, at least 45% sequence identity to SEQ ID NO: 133, at least 50% sequence identity to SEQ ID NO: 134, at least 50% sequence identity to SEQ ID NO: 135, at least 30% sequence identity to SEQ ID NO: 136, at least 55% sequence identity to SEQ ID NO: 137, at least 40% sequence identity to SEQ ID NO: 138, at least 40% sequence identity to SEQ ID NO: 139, and

(v) a protease / peptidase or a variant thereof having at least 50% sequence identity to SEQ ID NO: 140, at least 50% sequence identity to SEQ ID NO: 141, at least 35% sequence identity to SEQ ID NO: 142, at least 40% sequence identity to SEQ ID NO: 143, at least 60% sequence identity to SEQ ID NO: 144, at least 35% sequence identity to SEQ ID NO: 145, at least 40% sequence identity to SEQ ID NO: 146, at least 30% sequence identity to SEQ ID NO: 147, at least 55% sequence identity to SEQ ID NO: 148, at least 55% sequence identity to SEQ ID NO: 149, at least 30% sequence identity to SEQ ID NO: 150, at least 30% sequence identity to SEQ ID NO: 151, at least 30% sequence identity to SEQ ID NO: 152, at least 30% sequence identity to SEQ ID NO: 153, at least 50% sequence identity to SEQ ID NO: 154, at least 50% sequence identity to SEQ ID NO: 155, at least 55% sequence identity to SEQ ID NO: 156, at least 40% sequence identity to SEQ ID NO: 157, at least 40% sequence identity to SEQ ID NO: 158, at least 50% sequence identity to SEQ ID NO: 159, at least 50% sequence identity to SEQ ID NO: 160, at least 45% sequence identity to SEQ ID NO: 161, at least 45% sequence identity to SEQ ID NO: 162, at least 65% sequence identity to SEQ ID NO: 163, at least 55% sequence identity to SEQ ID NO: 164, at least 30% sequence identity to SEQ ID NO: 165, at least 55% sequence identity to SEQ ID NO: 166, at least 45% sequence identity to SEQ ID NO: 167, at least 60% sequence identity to SEQ ID NO: 168, at least 55% sequence identity to SEQ ID NO: 169, at least 50% sequence identity to SEQ ID NO: 170, at least 45% sequence identity to SEQ ID NO: 171, at least 50% sequence identity to SEQ ID NO: 172, at least 50% sequence identity to SEQ ID NO: 173, at least 40% sequence identity to SEQ ID NO: 174, at least 45%

sequence identity to SEQ ID NO: 175, at least 30% sequence identity to SEQ ID NO: 176, at least 50% sequence identity to SEQ ID NO: 177, at least 40% sequence identity to SEQ ID NO: 178, at least 45% sequence identity to SEQ ID NO: 179, at least 35% sequence identity to SEQ ID NO: 180, at least 55% sequence identity to SEQ ID NO: 181, at least 35% sequence identity to SEQ ID NO: 182, at least 50% sequence identity to SEQ ID NO: 183, at least 40% sequence identity to SEQ ID NO: 184, at least 30% sequence identity to SEQ ID NO: 185, at least 30% sequence identity to SEQ ID NO: 186, at least 55% sequence identity to SEQ ID NO: 187, at least 60% sequence identity to SEQ ID NO: 188, at least 50% sequence identity to SEQ ID NO: 189, at least 35% sequence identity to SEQ ID NO: 190, and
(vi) a mixture thereof; and

(b) at least one cleaning component preferably selected from a surfactant, a builder, a bleach component, a polymer, a dispersing agent and an additional enzyme.

**2.** A composition according to claim 1 comprising a glycosyl hydrolase wherein the glycosyl hydrolase is a laminarinase, preferably an endo-beta-1,3-glucanase (E.C. 3.2.1.39).

**3.** A composition according to claim 1 comprising a glycosyl hydrolase selected from the group consisting of glycosyl hydrolases having at least 35%, preferably at least 45% sequence identity to SEQ ID NO: 1, at least 45%, preferably at least 55% sequence identity to SEQ ID NO: 2, at least 55%, preferably at least 65% sequence identity to SEQ ID NO: 3, at least 55%, preferably at least 65% sequence identity to SEQ ID NO: 49, at least 65%, preferably at least 75% sequence identity to SEQ ID NO: 54, at least 60%, preferably at least 70% sequence identity to SEQ ID NO: 58, at least 70%, preferably at least 80% sequence identity to SEQ ID NO: 59, at least 60%, preferably at least 70% sequence identity to SEQ ID NO: 60, at least 45%, preferably at least 55% sequence identity to SEQ ID NO: 71, at least 45%, preferably at least 55% sequence identity to SEQ ID NO: 93, at least 50%, preferably at least 60% sequence identity to SEQ ID NO: 94, at least 55%, preferably at least 65% sequence identity to SEQ ID NO: 95, at least 30%, preferably at least 40% sequence identity to SEQ ID NO: 101 and a mixture thereof.

**4.** A composition according to the preceding claim wherein the glycosyl hydrolase is selected from the group consisting of glycosyl hydrolases having at least 55%, preferably at least 85% sequence identity to SEQ ID NO: 1, at least 65%, preferably at least 85% sequence identity to SEQ ID NO: 2, at least 75%, preferably at least 90% sequence identity to SEQ ID NO: 3, at least 65%, preferably at least 85% sequence identity to SEQ ID NO: 49, at least 75%, preferably at least 90% sequence identity to SEQ ID NO: 54, at least 70%, preferably at least 90% sequence identity to SEQ ID NO: 58, at least 80%, preferably at least 95% sequence identity to SEQ ID NO: 59, at least 70%, preferably at least 90% sequence identity to SEQ ID NO: 60, at least 65%, preferably at least 85% sequence identity to SEQ ID NO: 71, at least 65%, preferably at least 85% sequence identity to SEQ ID NO: 93, at least 70%, preferably at least 95% sequence identity to SEQ ID NO: 94, at least 75%, preferably at least 95% sequence identity to SEQ ID NO: 95, at least 50%, preferably at least 80% sequence identity to SEQ ID NO: 101 and a mixture thereof.

**5.** A composition according to the preceding claim wherein the glycosyl hydrolase is selected from the group consisting of glycosyl hydrolases having at least 95%, preferably at least 98% sequence identity to a sequence selected from the group consisting of SEQ ID NO: 1-3, 39, 49, 54, 58-60, 71, 93-95, 101 and a mixture thereof.

**6.** A composition according to any of the preceding claims comprising a glycosyl hydrolase of SEQ ID NO: 1

**7.** A composition according to any of the preceding claims wherein the composition is a laundry detergent composition or an automatic dishwashing composition.

**8.** A composition according to any of the preceding claims wherein the composition has a pH of greater than 7.5, preferably from 8 to 11.5, as measured in a 1% by weight aqueous solution at 25°C.

**9.** A composition according to any of the preceding claims wherein the composition comprises an additional enzyme, preferably an amylase and a protease.

**10.** A composition according to any of the preceding claims wherein the composition comprises a surfactant.

**11.** A composition according to any of the preceding claims wherein the composition is a laundry composition comprising:

a) from about to about 1 to about 40% by weight of the composition of a surfactant, preferably an anionic

surfactant;
b) from 0.00005 to 5% by weight of the composition of an additional enzyme comprising an amylase and a protease and optionally a lipase;
c) from about to about 10 to 60% by weight of the composition of a builder, preferably a builder selected from builders based on carbonate, bicarbonate or silicates; and
d) optionally a soil release polymer.

**12.** A composition according to any of the preceding claims wherein the composition is an automatic dishwashing composition comprising:

a) from about to about 1 to 10% by weight of the composition of a surfactant, preferably non-nionic surfactant;
b) from 0.00005 to 5% by weight of the composition of an additional enzyme comprising an amylase and a protease;
c) from about to about 5 to 50% by weight of the composition of a builder, preferably selected from citrate, a salt of methyl glycine diacetic acid and a mixture thereof; and
d) optionally a polymer, preferably a sulfonate polymer.

**13.** A method of cleaning a substrate comprising the step of contacting the substrate with a composition according to any of the preceding claims and optionally rinsing the substrate.

**14.** Use of an enzyme endogenous to *Alkalitalea saponilacus* or a variant thereof as defined in claim 1 in an alkaline cleaning composition to provide cosmetic stain removal.

**15.** Use of an enzyme of SEQ ID NO: 1 or a variant having at least 35%, preferably at least 50% identity to SEQ ID NO: 1 in an alkaline cleaning composition to provide cosmetic stain removal from fabrics.

Europäisches Patentamt
European Patent Office
Office européen des brevets

# EUROPEAN SEARCH REPORT

Application Number
EP 23 18 0130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GenPept [Online]<br><br>16 May 2022 (2022-05-16),<br>"family 16 glycosylhydrolase [Alkalitalea saponilacus].",<br>XP002810418,<br>retrieved from https://www.ncbi.nlm.nih.gov/protein/1160696343?sat=5&satkey=781594223<br>Database accession no. WP_079556612<br>* the whole document *<br>----- | 1-15 | INV.<br>C11D3/386 |
| A | ZHAO BAISUO ET AL: "Alkalitalea saponilacus gen. nov., sp. nov., an obligately anaerobic, alkaliphilic, xylanolytic bacterium from a meromictic soda lake",<br>INTERNATIONAL JOURNAL OF SYSTEMATIC AND EVOLUTIONARY MICROBIOLOGY,<br>vol. 62, no. Pt_11,<br>1 November 2012 (2012-11-01), pages 2618-2623, XP93097837,<br>GB<br>ISSN: 1466-5026, DOI: 10.1099/ijs.0.038315-0<br>-----<br>-/-- | 1 | |
| | | | TECHNICAL FIELDS SEARCHED (IPC) |
| | | | C11D |

~~The present search report has been drawn up for all claims~~

2

| Place of search | Date of completion of the search | Examiner |
|---|---|---|
| The Hague | 13 February 2024 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 0130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIAO ZIYA ET AL: "Insights into Xylan Degradation and Haloalkaline Adaptation through Whole-Genome Analysis of Alkalitalea saponilacus, an Anaerobic Haloalkaliphilic Bacterium Capable of Secreting Novel Halostable Xylanase", GENES, vol. 10, no. 1, 20 December 2018 (2018-12-20), page 1, XP93097903, US ISSN: 2073-4425, DOI: 10.3390/genes10010001 ----- | 1 | |
| A | WO 2020/201403 A1 (NOVOZYMES AS [DK]) 8 October 2020 (2020-10-08) * the whole document * ----- | 1-15 | |
| A | WO 2020/186028 A1 (PROCTER & GAMBLE [US]) 17 September 2020 (2020-09-17) ----- | 1 | |
| X | DATABASE EMBL [Online] 3 July 2018 (2018-07-03), "Alkalitalea saponilacus beta-mannosidase ID - ASB49906; SV 1; linear; genomic DNA; STD; PRO; 1146 BP.", XP002811002, retrieved from EBI accession no. EMBL:ASB49906 * the whole document * ----- | 1-5,7-14 | TECHNICAL FIELDS SEARCHED (IPC) |

-/--

~~The present search report has been drawn up for all claims~~

2

| Place of search | Date of completion of the search | Examiner |
|---|---|---|
| The Hague | 13 February 2024 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 0130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online]<br><br>3 July 2018 (2018-07-03), "Alkalitalea saponilacus peptidase M23 ID - ASB47742; SV 1; linear; genomic DNA; STD; PRO; 1734 BP.", XP002811003, retrieved from EBI accession no. EMBL:ASB47742<br>* the whole document *<br>----- | 1,7-14 | |
| A | UMA GANAPATHI ET AL: "Nature and bioprospecting of haloalkaliphilics: a review", WORLD JOURNAL OF MICROBIOLOGY AND BIOTECHNOLOGY, SPRINGER NETHERLANDS, DORDRECHT, vol. 36, no. 5, 23 April 2020 (2020-04-23), XP037099018, ISSN: 0959-3993, DOI: 10.1007/S11274-020-02841-2 [retrieved on 2020-04-23]<br>----- | 1 | TECHNICAL FIELDS SEARCHED (IPC) |

2

~~The present search report has been drawn up for all claims~~

| Place of search | Date of completion of the search | Examiner |
|---|---|---|
| The Hague | 13 February 2024 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 23 18 0130

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [x] Only part of the further search fees have been paid within the fixed time limit. The present European search report has been drawn up for those parts of the European patent application which relate to the inventions in respect of which search fees have been paid, namely claims:

  **1-15(partially)**

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 23 18 0130

The Search Division considers that the present European patent application does not comply with the requirements of unity of invention and relates to several inventions or groups of inventions, namely:

1. claims: 6, 15(completely); 1-5, 7-14(partially)

An alkaline cleaning composition comprising 0.00005-5% of a glycosyl hydrolase having at least 35% sequence identity to SEQ.ID.NO:1 and at least one cleaning component

---

2-105. claims: 1-5, 7-14(all partially)

Subject matter analogous to invention 1, but limited to the respective glycosyl hydrolases having the respective percentages of sequence identity as listed in claim 1 to the respective SEQ.ID.NO's 2-105.

---

106-111. claims: 1, 7-14(all partially)

Subject matter analogous to invention 1, but limited to the respective polysaccharide lyases having the respective percentages of sequence identity as listed in claim 1 to the respective SEQ.ID.NO's 106-111.

---

112-124. claims: 1, 7-14(all partially)

Subject matter analogous to invention 1, but limited to the respective esterases / lipases having the respective percentages of sequence identity as listed in claim 1 to the respective SEQ.ID.NO's 112-124.

---

125-139. claims: 1, 7-14(all partially)

Subject matter analogous to invention 1, but limited to the respective nucleases having the respective percentages of sequence identity as listed in claim 1 to the respective SEQ.ID.NO's 125-139.

---

140-190. claims: 1, 7-14(all partially)

Subject matter analogous to invention 1, but limited to the respective proteases / peptidases having the respective percentages of sequence identity as listed in claim 1 to the respective SEQ.ID.NO's 140-190, cleaning method, use in an alkaline cleaning composition to provide cosmetic stain removal

---

## ANNEX TO THE EUROPEAN SEARCH REPORT ON EUROPEAN PATENT APPLICATION NO. EP 23 18 0130

This annex lists the patent family members relating to the patent documents cited in the above-mentioned European search report. The members are as contained in the European Patent Office EDP file on
The European Patent Office is in no way liable for these particulars which are merely given for the purpose of information.

13-02-2024

| Patent document cited in search report | Publication date | Patent family member(s) | Publication date |
|---|---|---|---|
| WO 2020201403 A1 | 08-10-2020 | CN 113785039 A | 10-12-2021 |
| | | EP 3947619 A1 | 09-02-2022 |
| | | US 2022169953 A1 | 02-06-2022 |
| | | WO 2020201403 A1 | 08-10-2020 |
| WO 2020186028 A1 | 17-09-2020 | CA 3127167 A1 | 17-09-2020 |
| | | CN 113439116 A | 24-09-2021 |
| | | EP 3938484 A1 | 19-01-2022 |
| | | JP 7275297 B2 | 17-05-2023 |
| | | JP 2022522852 A | 20-04-2022 |
| | | US 2020291332 A1 | 17-09-2020 |
| | | WO 2020186028 A1 | 17-09-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004067737 A
- WO 2015091989 A
- WO 2015091990 A
- WO 2015024739 A
- WO 2015143360 A
- US 6312936 B1
- US 5679630 A
- US 4760025 A
- DE 102006022216 A1
- DE 102006022224 A1
- WO 2015089447 A
- WO 2015089441 A
- WO 2016066756 A
- WO 2016066757 A
- WO 2016069557 A
- WO 2016069563 A
- WO 2016069569 A
- WO 2016174234 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- WO 0037627 A
- WO 08010925 A
- US 5352604 A
- US 6939702 B1
- US 20090217464 A
- WO 2013171241 A
- WO 2011084412 A
- WO 2013033318 A
- WO 2018228880 A
- WO 2018228881 A
- EP 3299457 A
- WO 2018209026 A
- WO 2017036901 A
- WO 2017005798 A
- US 4435307 A
- US 5648263 A
- US 5691178 A
- US 5776757 A
- US 7141403 B2
- WO 09148983 A
- WO 2017106676 A
- US 7153818 B
- WO 9700324 A
- EP 1022334 A
- US 5856164 A
- WO 9923211 A
- WO 9623873 A
- WO 0060060 A
- WO 06002643 A
- WO 2017192657 A
- WO 2011100410 A
- WO 2013003659 A
- US 6093562 A
- WO 09149130 A
- WO 10115021 A
- WO 2016091688 A
- WO 2014099523 A
- WO 2014164777 A
- WO 2009149271 A
- WO 2016180748 A
- WO 2018060216 A
- WO 9324618 A
- WO 9510602 A
- WO 9815257 A
- WO 2018191135 A
- WO 2015040159 A
- WO 2017021515 A
- WO 2017021516 A
- WO 2017021517 A
- WO 2019081515 A
- WO 2017162836 A
- WO 2018108865 A
- WO 2018011277 A
- EP 3476935 A
- WO 2018178061 A
- WO 2020074499 A
- WO 2018184873 A
- WO 2017186936 A
- WO 2017186937 A
- WO 2017186943 A
- WO 2017207770 A
- WO 2019086520 A
- WO 2019086528 A
- WO 2019086530 A
- WO 2019086532 A
- WO 2019086521 A
- WO 2019086526 A
- WO 2020002604 A
- WO 2020002608 A
- WO 2020007863 A

- WO 2020007875 A
- WO 2020008024 A
- WO 2020070063 A
- WO 2020070249 A
- WO 2020088957 A
- WO 2020088958 A
- WO 2020207944 A
- WO 2022094164 A
- WO 2019228448 A
- WO 2018102478 A
- WO 2018185181 A
- WO 2020070011 A
- WO 2018185267 A
- WO 2020070209 A
- WO 2020008043 A
- WO 2018102479 A
- WO 2018185280 A
- WO 2022094588 A
- WO 2022094163 A
- WO 2013167581 A
- WO 2015181299 A
- WO 2015181292 A
- WO 2017046232 A
- WO 2017046260 A
- WO 201837062 A
- WO 201837065 A
- WO 2019038059 A
- WO 2019162000 A
- WO 2015001017 A
- WO 2018037061 A
- WO 201837064 A
- WO 2019038060 A
- WO 2019038057 A
- WO 2015185689 A
- EP 1794275 A
- EP 1794276 A
- US 7208459 B2
- WO 201198355 A
- WO 201147987 A
- US 2012090102 A
- WO 2010145887 A
- WO 2006055787 A
- WO 2010142503 A
- US 20080177090 A
- WO 2011011799 A
- US 8138222 B
- WO 09118375 A
- WO 9813459 A
- US 6020303 A
- US 6060443 A
- US 8765659 B

**Non-patent literature cited in the description**


- **NEEDLEMAN** ; **WUNSCH**. *J. Mol. Biol.*, 1970, vol. 48, 443-453
- **RICE et al.** *EMBOSS: The European Molecular Biology Open Software Suite*, 2000
- *Trends Genet.*, vol. 16, 276-277
- Enzymes, Detergent.. **FLICKINGER, M.C.** ; **MAURER, K.-H.** Encyclopaedia of Industrial Biotechnology. 2010
- **OLSEN, H.S.** ; **FALHOLT, P.** The role of enzymes in modern detergency.. *J Surfact Deterg*, 1998, vol. 1, 555-567
- **LI XL** ; **ZHANG WH** ; **WANG YD** ; **DAI YJ** ; **ZHANG HT** ; **WANG Y** ; **WANG HK** ; **LU FP**. A high-detergent-performance, cold-adapted lipase from Pseudomonas stutzeri PS59 suitable for detergent formulation. *J Mol Catal B Enzym*, 2014, vol. 102, 16-24
- **MYKYTCZUK, N.** ; **FOOTE, S.** ; **OMELON, C. et al.** Bacterial growth at -15 °C; molecular insights from the permafrost bacterium Planococcus halocryophilus Or1. *ISME J*, 2013, vol. 7, 1211-1226
- **AL-GHANAYEM, A.A.** ; **JOSEPH, B.** Current prospective in using cold-active enzymes as eco-friendly detergent additive. *Appl Microbiol Biotechnol*, 2020, vol. 104, 2871-2882
- **JI X** ; **CHEN G** ; **ZHANG Q** ; **LIN L**. Purification and characterization of an extracellular cold-adapted alkaline lipase produced by psychrotrophic bacterium Yersinia enterocolitica strain KM1. *J Basic Microbiol*, 2015, vol. 55 (6), 718-728
- **ZHAO B** ; **CHEN S**. Alkalitalea saponilacus gen. nov., sp. nov., an obligately anaerobic, alkaliphilic, xylanolytic bacterium from a meromictic soda lake. *Int J Syst Evol Microbiol*, 2012, vol. 62, 2618-2623
- **DARTOIS et al.** *Biochemica et Biophysica Acta*, 1993, vol. 1131, 253-360